# EUROPEAN PATENT APPLICATION

(11) **EP 4 438 653 A1**
(43) Date of publication of application: **02.10.2024**
(21) Application number: 22898524.8
(22) Date of filing: 21.11.2022
(51) Int. Cl.: C08G 65/32, A61K 38/13, A61K 39/395, A61K 47/59

(54) **FOUR-BRANCH TYPE WATER SOLUBLE POLYMER FOR MEDICAL USE**

(30) Priority: 25.11.2021 JP 2021191074
(71) Applicant: NOF Corporation, Shibuya-ku Tokyo 150-6019 (JP)
(72) Inventor: HAMURA, Ken, Kawasaki-shi, Kanagawa 210-0865 (JP); YOSHIOKA, Hiroki, Kawasaki-shi, Kanagawa 210-0865 (JP); KAMIYA, Masaki, Kawasaki-shi, Kanagawa 210-0865 (JP)
(74) Representative: Weickmann & Weickmann PartmbB
(86) International application number: PCT/JP2022/042986
(87) International publication number: WO 2023/095741

(57) **Abstract**

The present invention provides four-branched water-soluble polymers for medical use that are represented by the following formula (1) or the formula (2) (the symbols in the following formulas are as defined in the specification.

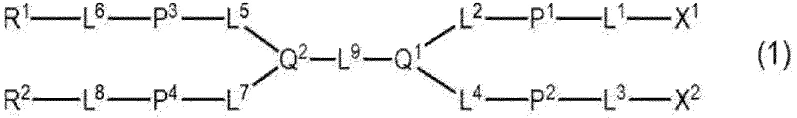

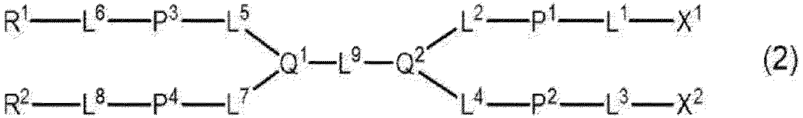

## Description

### [Technical Field]

The present invention relates to four-branched water-soluble polymers for medical use that improve solubility of conjugates with biologically relevant materials and can suppress aggregation of the aforementioned conjugates.

### [Background Art]

When pharmaceutical products using biologically relevant materials such as antibody, hormone, cytokine, enzyme, and the like are administered into the body, they are generally excreted rapidly from the body through glomerular filtration in the kidney and uptake by macrophages in the liver, spleen, and the like. Therefore, the half-life thereof in the blood is short, and it is often difficult to obtain sufficient pharmacological effects. To solve this problem, attempts have been made to chemically modify biologically relevant materials with water-soluble polymers such as polyethylene glycol (PEG), albumin, and the like. As a result, it becomes possible to extend the blood half-life of biologically relevant materials by increasing the molecular weight, forming a hydrated layer, and the like. It is also well known that these modifications can achieve effects such as reducing the toxicity and antigenicity of biologically relevant materials and suppressing aggregation thereof.

Antibodies, which are one of biologically relevant substances, have high binding affinity, binding specificity, and high stability in blood, and are currently applied to many diagnostic agents and pharmaceutical products. However, since Escherichia coli, which is widely used as a means of producing general biologically relevant materials, does not have a glycosylation function, most antibody drugs are currently produced using mammalian cells that add human-type sugar chains (CHO cells, NSO cells, etc.). The sugar chains of antibodies produced by CHO cells, and the like are biosynthesized by sugar chain transferases. Thus, it is known that the sugar chain structure and the amount of glycosylation vary even in the same cell line due to different passages. Even if the produced antibodies are uniform at the amino acid sequence level, they are problematically heterogeneous at the sugar chain level (Non Patent Literature 1). In addition, antibodies pose problems that they require higher production costs because animal cells are used rather than inexpensive E. coli, and they also tend to aggregate easily (Non Patent Literature 2).

Furthermore, antibodies are large molecules with a molecular weight of about 160,000, and even though they have a long half-life in blood, they have the problem of extremely slow transfer from the blood into tissues. Therefore, studies are being conducted on antibody fragments that are obtained by reducing the molecular weight of antibody and increasing biotransferability. Antibody fragments have small molecular weights and do not have sugar chains. Thus, unlike antibodies, they can be produced in Escherichia coli, which is advantageous in terms of production costs.

As antibody fragments having antibody variable regions, various types such as Fv, Fab, Fab', F(ab')₂, single chain antibody (scFv), bispecific antibody (diabody), animal-derived nanobody, and the like are known. They are associated with a problem that low molecular weights cause shortening of the half-life in blood. As a method for solving this problem, antibody fragment conjugates modified with water-soluble polymers such as PEG have been provided (Patent Literature 1, Patent Literature 2). The half-life in blood can be adjusted by increasing the molecular weight of PEG used for modification, and in the case of antibody fragments, the half-life in blood can be extended by binding to PEG with a molecular weight of 40 kDa (Non Patent Literature 3). However, in some cases, these PEGylated antibody fragments also caused aggregation, similar to antibodies.

In addition, it is known that antibody fragments modified with such water-soluble polymers have reduced binding affinity to the target. Therefore, water-soluble polymers that can maintain binding affinity, suppress aggregation, and extend half-life in blood are demanded.

Some of peptides, which are among biologically relevant materials, have high binding affinity and high binding specificity similar to those of antibodies. In particular, efficient screening methods have been developed for cyclic peptides actively developed in recent years, and it is known that incorporating unnatural amino acids can impart protease resistance and high binding affinity comparable to that of antibodies (Non Patent Literature 4).

Cyclic peptides have lower solubility than general peptides and are known to cause aggregation due to insolubilization and self-association, and efforts are being made to add solubilizers and the like or modify them with water-soluble polymers (Patent Literature 3, Non Patent Literature 5). Furthermore, in recent years, enhancement of pharmacological activity has been demanded in order to obtain the best therapeutic effect, and in Patent Literature 3, efforts are being made to load two or more molecules of peptide via a water-soluble polymer, as one way to enhance pharmacological activity.

However, compounds that carry two molecules of peptide via a water-soluble polymer with a linear structure and compounds that carry four molecules of peptide via a four-branched water-soluble polymer have been reported to self-associate and aggregate in an aqueous solution, and it is obvious that insolubilization and aggregation are promoted when two or more molecules of cyclic peptide are loaded via a conventional water-soluble polymer (Non Patent Literature 6). Therefore, a water-soluble polymer that can carry two or more molecules of a biologically relevant material is demanded in order to enhance pharmacological activity while suppressing insolubilization and aggregation.

### [Citation List]

### [Patent Literature]

[Patent Literature 1]
   JP 2010-522141 A
[Patent Literature 2]
   JP 4108760 B
[Patent Literature 3]
   JP 2020-500157 A

### [Non Patent Literature]

[Non Patent Literature 1]
   Biotechnol. Bioeng. 2004, 87, 614-622
[Non Patent Literature 2]
   Antibodies 2016, 5, 19
[Non Patent Literature 3]
   Bioconjugate Chem. 2012, 23, 1452-1462
[Non Patent Literature 4]
   Front Chem. 2020; 8: 447
[Non Patent Literature 5]
   Journal of Nanomaterials. 2017, 4, 16
[Non Patent Literature 6]
   Biomacromolecules. 2014, 15, 1543-1559

### [Summary of Invention]

### [Technical Problem]

The problem to be solved by the present invention is to provide a four-branched water-soluble polymer for medical use, that can suppress aggregation and insolubilization that occur when bonded to two molecules of biologically relevant materials.

### [Solution to Problem]

In order to solve the above-mentioned problems, the present inventors conducted intensive studies and, as a result, developed a four-branched water-soluble polymer for medical use that has four independent divalent groups of water-soluble polymers in one molecule, in which two of the divalent groups of water-soluble polymers have a functional group, capable of reacting with a physiologically relevant material, at the ends of the divalent groups via a single bond or spacer. Accordingly, the present invention provides the following.
[1] A four-branched water-soluble polymer for medical use, represented by the formula (1) or the formula (2): 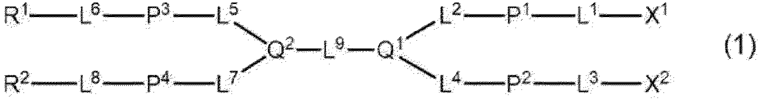 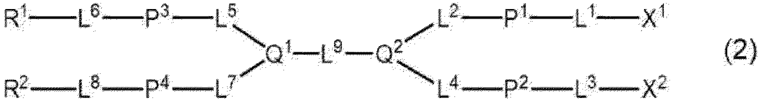 wherein
   X¹ and X² are each independently a functional group capable of reacting with a biologically relevant material,
   P¹ to P⁴ are each independently a divalent group of a water-soluble polymer,
   Q¹ is a trivalent group represented by the formula (q1): 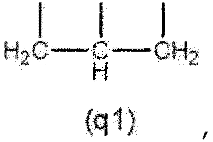
   Q² is a trivalent group represented by any of the formula (q1) to the formula (q3): 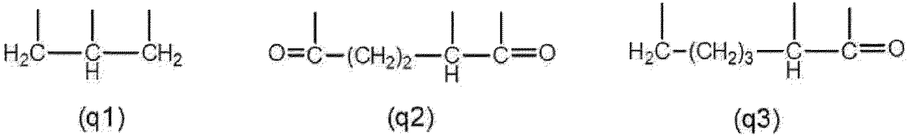
   L¹ to L⁸ are each independently a single bond or a divalent spacer,
   L⁹ is a divalent spacer, and
   R¹ and R² are each independently a hydrocarbon group.
[2] A four-branched water-soluble polymer for medical use, represented by the formula (3) or the formula (4): 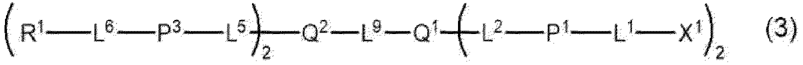 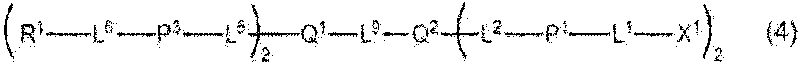 wherein
   X¹ is a functional group capable of reacting with a biologically relevant material,
   P¹ and P³ are each independently a divalent group of a water-soluble polymer,
   Q¹ is a trivalent group represented by the formula (q1): 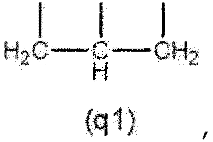
   Q² is a trivalent group represented by any of the formula (q1) to the formula (q3): 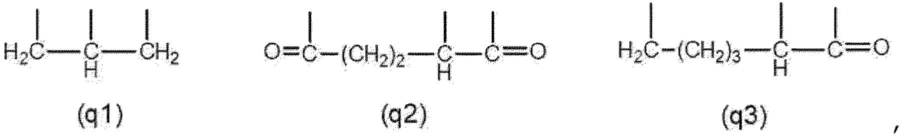
   L¹, L², L⁵, and L⁶ are each independently a single bond or a divalent spacer,
   L⁹ is a divalent spacer, and
   R¹ is a hydrocarbon group.
[3] The four-branched water-soluble polymer for medical use of the aforementioned [1] or [2], wherein the divalent groups of the water-soluble polymers are each independently a divalent group of polyethylene glycol, polyoxazoline, or polysialic acid.
[4] The four-branched water-soluble polymer for medical use of any one of the aforementioned [1] to [3], wherein a total of the number average molecular weight of the divalent groups of the water-soluble polymers is not less than 4,000 and not more than 160,000.
[5] The four-branched water-soluble polymer for medical use of any one of the aforementioned [1] to [4], wherein the functional group capable of reacting with a biologically relevant material is an active ester group, an active carbonate group, a formyl group, an isocyanate group, an isothiocyanate group, an epoxy group, a maleimidyl group, a vinylsulfonyl group, an acryloyl group, an alkylsulfonyloxy group, a carboxy group, a mercapto group, a pyridyldithio group, an α-haloacetyl group, an alkynyl group, an allyl group, a vinyl group, an amino group, an aminooxy group, a hydrazide group, or an azido group.

### [Advantageous Effects of Invention]

The four-branched water-soluble polymer for medical use of the present invention can improve the problems, aggregation and solubility, which have been associated with antibodies and cyclic peptides (biologically relevant materials). Furthermore, since the four-branched water-soluble polymer for medical use of the present invention has two functional groups capable of reacting with a biologically relevant material in one molecule, a conjugate of the four-branched water-soluble polymer for medical use of the present invention and two biologically relevant materials may have improved pharmacological activity as compared with conventional conjugates of a water-soluble polymer and one biologically relevant material.

### [Description of Embodiments]

The present invention is explained in detail in the following.

Only one kind of the four-branched water-soluble polymer for medical use of the present invention (hereinafter sometimes to be abbreviated as a "water-soluble polymer of the present invention") may be used, or two or more kinds thereof may be used in combination. The water-soluble polymer of the present invention is represented by the following formula (1) or the formula (2). In the present specification, the end of the straight line "-" in the chemical formula representing a group (e.g., the upper end of the straight line in the following formula (q1)) indicates a bonding position, not a carbon atom. 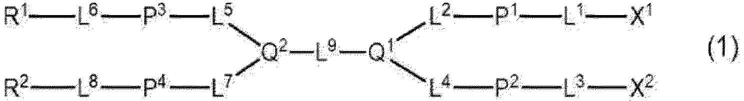 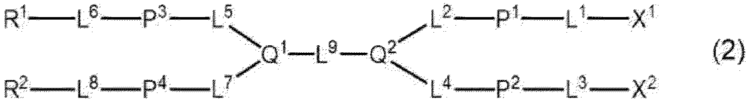 wherein
X¹ and X² are each independently a functional group capable of reacting with a biologically relevant material,
P¹ to P⁴ are each independently a divalent group of a water-soluble polymer,
Q¹ is a trivalent group represented by the formula (q1): 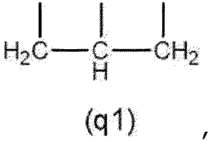
Q² is a trivalent group represented by any of the formula (q1) to the formula (q3): 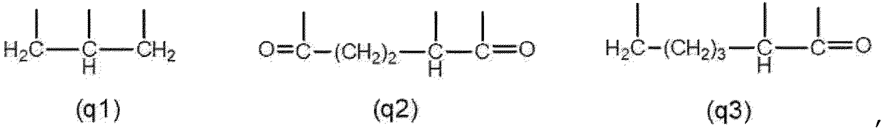
L¹ to L⁸ are each independently a single bond or a divalent spacer,
L⁹ is a divalent spacer, and
R¹ and R² are each independently a hydrocarbon group.

In the present specification, "the water-soluble polymer of the present invention represented by the formula (1)" is sometimes to be abbreviated as "water-soluble polymer (1)". The water-soluble polymers of the present invention represented by other formulas are sometimes abbreviated similarly. When the water-soluble polymer of the present invention falls under both the water-soluble polymer (1) and the water-soluble polymer (2), such water-soluble polymer is classified into the water-soluble polymer (1) in the present invention.

The water-soluble polymer of the present invention is preferably represented by the following formula (3) or formula (4). As used herein, the water-soluble polymer of the present invention represented by the formula (3) or formula (4) is a water-soluble polymer of the formula (1) or (2) wherein "X²-L³-P²-L⁴-" and "-L⁷-P⁴-L⁸-R²" are "X¹-L¹-P¹-L²-" and "-L⁵-P³-L⁶-R¹", respectively. The symbols in the formula (3) and the formula (4) are as defined above. Unless particularly indicated, the symbols in the formula (3) or the formula (4) are as described for the symbols in the below-mentioned formula (1) or the formula (2) (preferred embodiments, etc.).

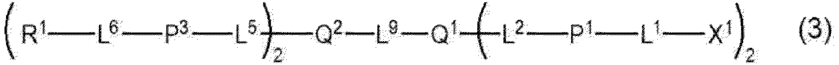

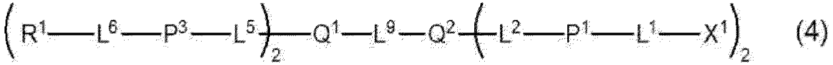

When the water-soluble polymer of the present invention falls under both the water-soluble polymer (3) and the water-soluble polymer (4), such water-soluble polymer is classified into the water-soluble polymer (3) in the present invention.

The groups in the formula (1) or the formula (2) are described sequentially in the following.

The divalent groups of the water-soluble polymers of P¹ to P⁴ are preferably each independently a divalent group of polyethylene glycol (PEG), polyoxazoline, or polysialic acid.

In the present specification, the divalent group of polyethylene glycol means a divalent group having a structure in which hydrogen atoms are removed from the hydroxy groups at the both ends of polyethylene glycol (i.e., divalent group represented by -O-(C₂H₄O)ₙ- wherein n is the number of repeat units).

In the present specification, the divalent group of polyoxazoline means a divalent group represented by the following formula: 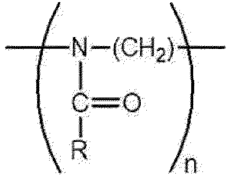 wherein R is an alkyl group, and n is the number of repeat units. R is preferably an alkyl group having 1 to 5 carbon atoms.

In the present specification, the divalent group of polysialic acid means a divalent group having a structure in which hydrogen atoms are removed from the hydroxy groups at the both ends of polysialic acid.

In one preferred embodiment of the present invention, the divalent groups of the water-soluble polymers of P¹ to P⁴ are each independently a divalent group of polyethylene glycol or polysialic acid, more preferably a divalent group of polyethylene glycol. When P¹ to P⁴ are divalent groups of polyethylene glycol, the number average molecular weight of the divalent groups of polyethylene glycol may be the same or different.

In both the formula (1) and the formula (2), the total of the number average molecular weights of the divalent groups of water-soluble polymers of P¹ to P⁴ is preferably not less than 4,000 and not more than 160,000, more preferably not less than 10,000 and not more than 120,000, further preferably not less than 20,000 and not more than 80,000. In one preferred embodiment of the present invention, in both the formula (1) and the formula (2) of the present invention, the total of the number average molecular weights of the water-soluble polymers of P¹ to P⁴ is not less than 20,000 and not more than 160,000. The number average molecular weight of the water-soluble polymer can be measured by gel permeation chromatography (GPC).

In both the formula (3) and the formula (4), the total of the number average molecular weights of the two P¹ and two P³ is preferably not less than 4,000 and not more than 160,000, more preferably not less than 10,000 and not more than 120,000, further preferably not less than 20,000 and not more than 80,000. In one preferred embodiment of the present invention, in both the formula (3) and the formula (4) of the present invention, the total of the number average molecular weights of the two P¹ and two P³ is not less than 20,000 and not more than 160,000.

The number average molecular weights of the divalent groups of water-soluble polymers of P¹ to P⁴ are each independently preferably 1,000 to 80,000, more preferably 2,000 to 60,000, further preferably 2,500 to 40,000.

Q¹ is a trivalent group represented by the formula (q1), and Q² is a trivalent group represented by any of the formula (q1) to the formula (q3). The trivalent group represented by the formula (q1) can be formed, for example, from glycerol or a derivative thereof. A trivalent group represented by the formula (q2) can be formed, for example, from glutamic acid or a derivative thereof. The trivalent group represented by the formula (q3) can be formed, for example, from lysine or a derivative thereof. Q² is preferably a trivalent group represented by the formula (q1) or the formula (q2), more preferably a trivalent group represented by the formula (q1).

X¹ and X² are each independently a functional group capable of reacting with a biologically relevant material. The functional group is not particularly limited as long as it is a functional group that reacts with a functional group present in biologically relevant materials such as bioactive protein, peptide, antibody, nucleic acid, and the like to be chemically modified to form a covalent bond. As the functional group, for example, the functional groups described in "Harris, J. M. Poly (Ethylene Glycol) Chemistry; Plenum Press: New York, 1992", "Hermanson, G. T. Bioconjugate Techniques, 2nd ed.; Academic Press: San Diego, CA, 2008", "PEGylated Protein Drugs: Basic Science and Clinical Applications; Veronese, F. M., Ed.; Birkhauser: Basel, Switzerland 2009", and the like can be mentioned.

The functional group capable of reacting with a biologically relevant material is not particularly limited as long as it is a functional group that can be chemically bonded to a functional group of a biologically relevant material such as amino group, mercapto group, formyl group, carboxyl group, unsaturated bond, azide group, and the like.

Examples of the functional group capable of reacting with a biologically relevant material include active ester group, active carbonate group, formyl group, isocyanate group (alias "isocyanato group"), isothiocyanate group (alias "isothiocyanato group"), epoxy group, carboxy group, mercapto group, maleimidyl group, substituted maleimidyl group, hydrazide group, pyridyldithio group, substituted sulfonate group, vinylsulfonyl group, amino group, aminooxy group (H₂N-O-), iodoacetamido group, alkylcarbonyl group, alkenyl group (e.g., allyl group, vinyl group), alkynyl group, azido group, acryloyl group, α-haloacetyl group, and the like.

In the present specification, the active ester group means an ester group having an alkoxy group with high elimination ability (i.e., alkoxycarbonyl group). As the alkoxy group with high elimination ability, for example, an alkoxy group derived from nitrophenol, N-hydroxysuccinimide, pentafluorophenol, and the like (i.e., a group having a structure in which a hydrogen atom of a hydroxy group or phenolic hydroxyl group is removed from the aforementioned compound) can be mentioned. The active ester group is preferably an ester group having an alkoxy group derived from N-hydroxysuccinimide (i.e., N-succinimidyl oxycarbonyl group).

In the present specification, the active carbonate group means a carbonate group having an alkoxy group with high elimination ability (i.e., alkoxycarbonyloxy group). As the alkoxy group with high elimination ability, for example, an alkoxy group derived from nitrophenol, N-hydroxysuccinimide, pentafluorophenol, and the like (i.e., a group having a structure in which a hydrogen atom of a hydroxy group or phenolic hydroxyl group is removed from the aforementioned compound) can be mentioned. The active carbonate group is preferably a carbonate group having an alkoxy group derived from nitrophenol or N-hydroxysuccinimide (i.e., nitrophenyloxycarbonyloxy group or succinimidyl oxycarbonyloxy group), more preferably a carbonate group having an alkoxy group derived from 4-nitrophenol or N-hydroxysuccinimide (i.e., 4-nitrophenyloxycarbonyloxy group or N-succinimidyl oxycarbonyloxy group).

In the present specification, the maleimidyl group means an N-maleimidyl group (i.e., 1-maleimidyl group) and the substituted maleimidyl group means a maleimidyl group in which a hydrocarbon group is bonded to one carbon atom of the double bond of the maleimidyl group. The aforementioned hydrocarbon group is preferably a hydrocarbon group having 1 to 5 carbon atoms, more preferably an alkyl group having 1 to 5 carbon atoms. Examples of the alkyl having 1 to 5 carbon atoms include methyl group, ethyl group, propyl group, isopropyl group, butyl group, tert-butyl group, and the like. The aforementioned hydrocarbon group is further preferably a methyl group or an ethyl group.

In the present specification, the substituted sulfonate group is a group represented by -O-(SO₂)-R (wherein R is a hydrocarbon group optionally containing a fluorine atom). As the hydrocarbon group optionally containing a fluorine atom, for example, a methyl group, an ethyl group, a propyl group, an isopropyl group, a butyl group, a tert-butyl group, a hexyl group, a nonyl group, a vinyl group, a phenyl group, a benzyl group, a 4-methylphenyl group, a trifluoromethyl group, a 2,2,2-trifluoroethyl group, a 4-(trifluoromethoxy)phenyl group, and the like can be mentioned, and it is preferably a methyl group, a vinyl group, a 4-methylphenyl group, or a 2,2,2-trifluoroethyl group. The substituted sulfonate group is preferably an alkylsulfonyloxy group.

The functional group capable of reacting with a biologically relevant material is preferably an active ester group, an active carbonate group, a formyl group, an isocyanate group, an isothiocyanate group, an epoxy group, a maleimidyl group, a substituted maleimidyl group, a vinylsulfonyl group, an acryloyl group, a substituted sulfonate group, a carboxy group, a mercapto group, a pyridyldithio group, an α-haloacetyl group, an alkynyl group, an allyl group, a vinyl group, an amino group, an aminooxy group, a hydrazide group, or an azido group.

The functional group capable of reacting with a biologically relevant material is more preferably an active ester group, an active carbonate group, a formyl group, a maleimidyl group, a substituted maleimidyl group, a carboxy group, an amino group, or an aminooxy group, further preferably an active ester group, an active carbonate group, a maleimidyl group, a substituted maleimidyl group, a carboxy group, an amino group, or an aminooxy group, particularly preferably an active ester group, a maleimidyl group, a substituted maleimidyl group, a carboxy group, or an amino group, most preferably an active ester group, a maleimidyl group, a carboxy group, or an amino group.

In one embodiment of the present invention, the functional group capable of reacting with a biologically relevant material is a functional group selected from the group consisting of the following group (I), group (II), group (III), group (IV), group (V), and group (VI).

### Group (I): functional group capable of reacting with amino group of biologically relevant material

Functional groups represented by the following formula (a), the formula (b), the formula (c), the formula (d), the formula (e), the formula (f), the formula (g), the formula (j), and the formula (k) can be mentioned.

### Group (II): functional group capable of reacting with mercapto group of biologically relevant material

Functional groups represented by the following formula (a), the formula (b), the formula (c), the formula (d), the formula (e), the formula (f), the formula (g), the formula (h), the formula (i), the formula (j), the formula (k), and the formula (l) can be mentioned.

### Group (III): functional group capable of reacting with formyl group of biologically relevant material

Functional groups represented by the following formula (h), the formula (m), the formula (n), and the formula (p) can be mentioned.

### Group (IV): functional group capable of reacting with carboxy group of biologically relevant material

Functional groups represented by the following formula (h), the formula (m), the formula (n), and the formula (p) can be mentioned.

### Group (V): functional group capable of reacting with unsaturated bond of biologically relevant material

Functional groups represented by the following formula (h), the formula (m), and the formula (o) can be mentioned.

### Group (VI): functional group capable of reacting with azido group of biologically relevant material

A functional group represented by the following formula (l) can be mentioned.

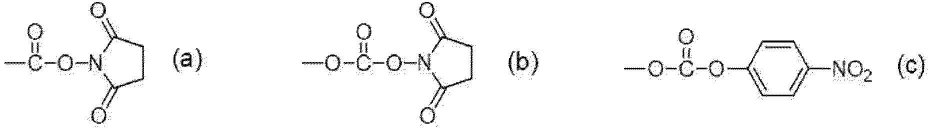

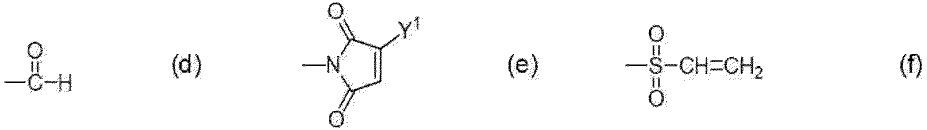

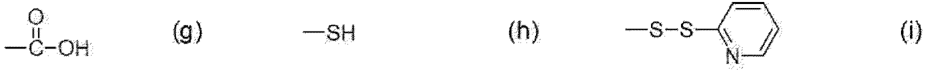

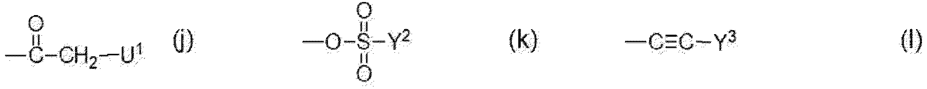

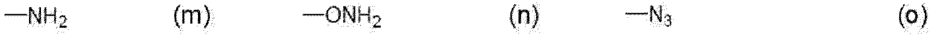

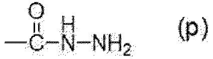

U¹ in the formula (j) is a halogen atom, preferably a chlorine atom (Cl), a bromine atom (Br), or a iodine atom (I), more preferably Br or I, further preferably I.

Y¹ in the formula (e) is a hydrogen atom or a hydrocarbon group having 1 to 5 carbon atoms, preferably a hydrogen atom or an alkyl group having 1 to 5 carbon atoms. Examples of the alkyl group having 1 to 5 carbon atoms include methyl group, ethyl group, propyl group, isopropyl group, butyl group, tert-butyl group, and the like. Y¹ is preferably a hydrogen atom, a methyl group, or an ethyl group, more preferably a hydrogen atom.

Y³ in the formula (l) is a hydrogen atom or an alkyl group having 1 to 5 carbon atoms, preferably an alkyl group having 1 to 5 carbon atoms. Specific examples of the alkyl group having 1 to 5 carbon atoms include methyl group, ethyl group, propyl group, isopropyl group, butyl group, tert-butyl group, and the like, and the alkyl group is preferably a methyl group or an ethyl group.

Y² in the formula (l) is a hydrocarbon group having 1 to 10 carbon atoms and optionally containing a hetero atom. Examples of the hetero atom include fluorine atom, oxygen atom, and the like. As the hydrocarbon group having 1 to 10 carbon atoms and optionally containing a hetero atom, for example, a methyl group, an ethyl group, a propyl group, an isopropyl group, a butyl group, a tert-butyl group, a hexyl group, a nonyl group, a vinyl group, a phenyl group, a benzyl group, a 4-methylphenyl group, a trifluoromethyl group, a 2,2,2-trifluoroethyl group, a 4-(trifluoromethoxy)phenyl group, and the like can be mentioned, and it is preferably a methyl group, a vinyl group, a 4-methylphenyl group, or a 2,2,2-trifluoroethyl group.

In one embodiment of the present invention, a functional group capable of reacting with a biologically relevant material is preferably a functional group represented by the formula (a) (i.e., N-succinimidyl oxycarbonyl group), a functional group represented by the formula (b) (i.e., N-succinimidyl oxycarbonyloxy group), a functional group represented by the formula (c) (i.e., 4-nitrophenyloxycarbonyloxy group), a functional group represented by the formula (e) (i.e., maleimidyl group or substituted maleimidyl group), a functional group represented by the formula (g) (i.e., carboxy group), a functional group represented by the formula (m) (i.e., amino group), or a functional group represented by the formula (n) (i.e., aminooxy group), more preferably an N-succinimidyloxycarbonyl group, a functional group represented by the formula (e), a carboxy group, or an amino group, further preferably an N-succinimidyloxycarbonyl group, a maleimidyl group, a carboxy group, or an amino group.

L¹ to L⁸ are each independently a single bond or a divalent spacer, and L⁹ is a divalent spacer.

The divalent spacers of L², L⁴, L⁵, and L⁷ are each independently preferably
(i) a degradable oligopeptide chain,
(ii) an alkylene group,
(iii) an amide bond (-NH-CO- or -CO-NH-),
(iv) an ether bond (-O-),
(v) a thioether bond (-S-),
(vi) a urethane bond (-NH-CO-O- or -O-CO-NH-),
(vii) an imino group (-NH-),
(viii) a carbonyl group (-CO-),
(ix) a urea bond (-NH-CO-NH-), or
(x) a combination of the above-mentioned (i) to (ix).

The degradable oligopeptide chain is not particularly limited as long as it is stable in the blood of a living body and has the ability to be degraded by intracellular enzymes. The degradable oligopeptide chain is preferably an oligopeptide chain composed of 2 to 5 residues of neutral amino acids excluding cysteine. Examples of the degradable oligopeptide chain include glycine-phenylalanine-leucine-glycine chain, glycine-glycine-phenylalanine-glycine chain, glycine-phenylalanine-glycine chain, glycine-leucine-glycine chain, valine-citrulline-glycine chain, valine-alanine-glycine chain, phenylalanine-glycine chain, and the like. The degradable oligopeptide chain is preferably a glycine-phenylalanine-leucine-glycine chain, a glycine-glycine-phenylalanine-glycine chain, a glycine-phenylalanine-glycine chain, a valine-citrulline-glycine chain, a valine-alanine-glycine chain, or a phenylalanine-glycine chain, more preferably a glycine-phenylalanine-leucine-glycine chain, a glycine-phenylalanine-glycine chain, a valine-citrulline-glycine chain, or a phenylalanine-glycine chain, further preferably a glycine-phenylalanine-leucine-glycine chain or a phenylalanine-glycine chain. The direction of the degradable oligopeptide chain is not particularly limited. In the case of a phenylalanine (Phe)-glycine (Gly) chain, for example, either -Gly-Phe- or -Phe-Gly-may be used.

The divalent spacers of L¹, L³, L⁶, L⁸, and L⁹ are each independently preferably
(i) an alkylene group,
(ii) an amide bond,
(iii) an ether bond,
(iv) a thioether bond,
(v) a urethane bond,
(vi) an imino group,
(vii) a carbonyl group,
(viii) a urea bond,
(ix) a combination of the above-mentioned (i) to (viii).

In one embodiment of the present invention, L¹ to L⁴, L⁶, and L⁸ are each independently a single bond (the following formula (z1) wherein s is 0) or a divalent spacer selected from group (VII) below, or a divalent spacer composed of a combination of two to four selected from group (VII), L⁵ and L⁷ are each independently a single bond (the following formula (z1) wherein s is 0) or a combination of a divalent spacer selected from the following group (VII) and a degradable oligopeptide chain, and L⁹ is a divalent spacer selected from the following group (VII) (s in the formula (z1) is an integer of one or more). A divalent spacer containing an ester bond and/or a carbonate bond is not preferred because it gradually decomposes in the blood of a living body.

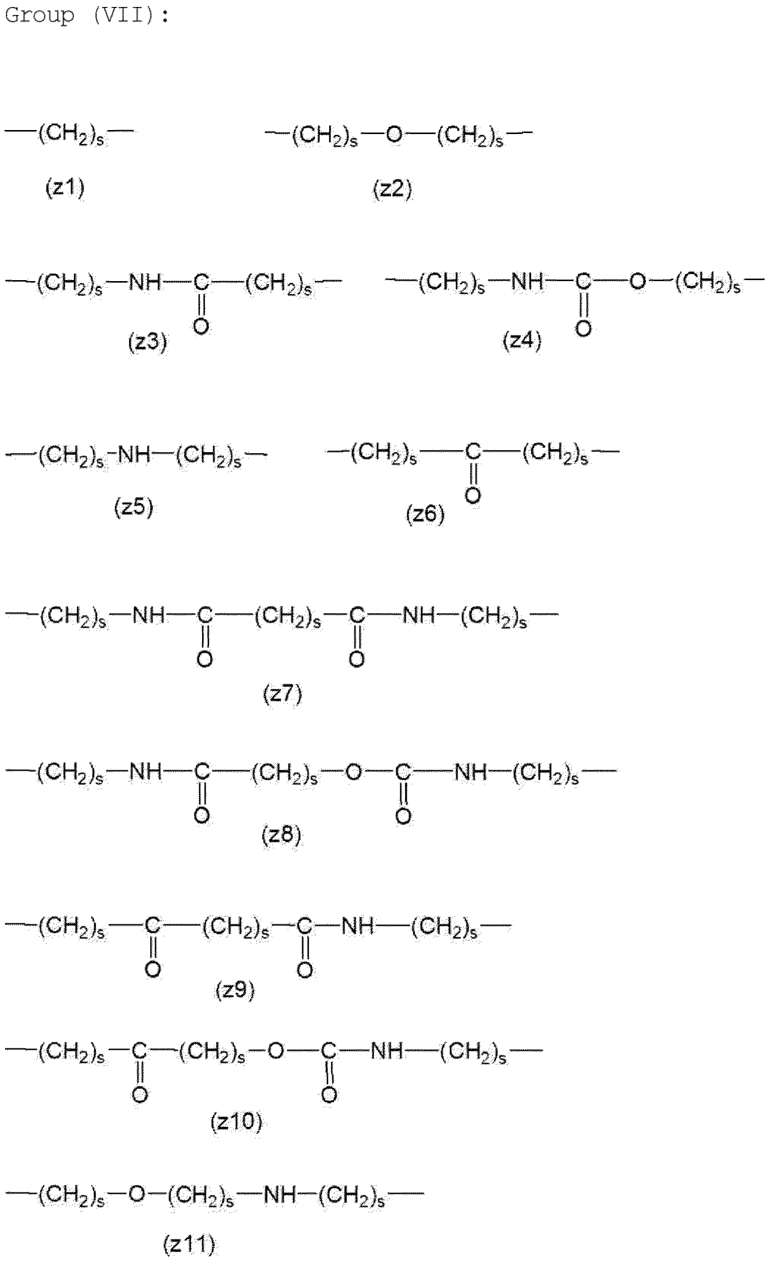

In the formula (z1) to the formula (z11), each s is independently an integer of 0 to 10, preferably an integer of 0 to 6, more preferably an integer of 0 to 3. When s is 0, -(CH₂)ₛ- in the above-mentioned formula is a single bond. Therefore, when s is 0, the formula (z1) is a single bond. In the formula (z2) to the formula (z11), a plurality of s in the same formula may be the same or different.

In group (VII), the direction of a left-right asymmetric chemical formula (e.g., the formula (z3)) is not particularly limited. For example, when L¹ is a divalent spacer represented by the formula (z3), the divalent spacer represented by the formula (z3) may bind to P¹ on the left side thereof and bind to X¹ or D¹ on the right side thereof, and may bind to X¹ or D¹ on the left side thereof and bind to P¹ on the right side thereof.

In one embodiment of the present invention, L¹ and L³ are each independently preferably a single bond or a divalent spacer represented by any of the formula (z12) to the formula (z14): 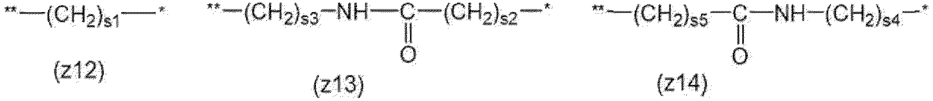 wherein s1 to s5 are each independently an integer of 0 to 10. When s1 is 0, -(CH₂)ₛ₁- is a single bond. When s2 to s5 are each 0 is the same as when s1 is 0. In the case of L¹, * in the formula (z12) to the formula (z14) shows a binding position to X¹, and ** shows a binding position to P¹. In the case of L², * in the formula (z12) to the formula (z14) shows a binding position to X², and ** shows a binding position to P².

s1 and s5 are each independently preferably an integer of 0 to 6, more preferably an integer of 0 to 3.

s2 to s4 are each independently preferably an integer of 0 to 6, more preferably an integer of 1 to 3.

In one embodiment of the present invention, L², L⁴, L⁶, and L⁸ are each preferably a single bond.

In one embodiment of the present invention, L⁹ is preferably a divalent spacer represented by the formula (z15) or the formula (z16): 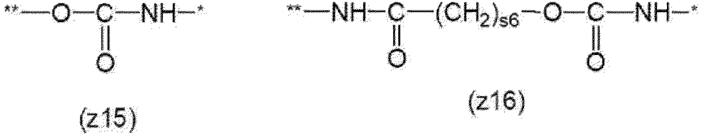 wherein s6 is an integer of 1 to 10. In addition, * in the formula (z15) and the formula (z16) shows a binding position to Q¹ and ** shows a binding position to Q².

s6 is preferably an integer of 1 to 6, more preferably an integer of 1 to 5.

In one embodiment of the present invention, L⁵ and L⁷ are each independently preferably a single bond or a divalent spacer represented by the formula (z17): 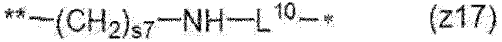 wherein s7 is an integer of 1 to 10, and L¹⁰ is a degradable oligopeptide chain. In the case of L⁵, * in the formula (z17) shows a binding position to Q¹ or Q², and ** shows a binding position to P³. In the case of L⁷, * in the formula (z17) shows a binding position to Q¹ or Q² and ** shows a binding position to P⁴.

s7 is preferably an integer of 1 to 6, more preferably an integer of 1 to 3.

L¹⁰ is preferably a phenylalanine-glycine chain, and it is more preferred that L¹⁰ is a phenylalanine-glycine chain, an imino group (-NH-) of -(CH₂)ₛ₇-NH- and a carbonyl group (-CO-) of the glycine residue in L¹⁰ are bonded, and an imino group (-NH-) of the phenylalanine residue in L¹⁰ are bonded to Q¹ or Q².

R¹ and R² are each independently a hydrocarbon group having 1 to 5 carbon atoms, preferably an alkyl group having 1 to 5 carbon atoms. Examples of the alkyl group having 1 to 5 carbon atoms include methyl group, ethyl group, propyl group, isopropyl group, butyl group, tert-butyl group, and the like. R¹ and R² are each independently more preferably a methyl group or an ethyl group.

Preferred examples of the water-soluble polymer (1) or water-soluble polymer (2) include the following.

### [Water-soluble polymer (1-1) or water-soluble polymer (2-1)]

Water-soluble polymer (1) or water-soluble polymer (2), wherein
X¹ and X² are each independently an active ester group, a maleimidyl group, a substituted maleimidyl group, a carboxy group, or an amino group,
P¹ to P⁴ are each independently a divalent group of polyethylene glycol or polysialic acid,
Q¹ is a trivalent group represented by the formula (q1),
Q² is a trivalent group represented by the formula (q1) or the formula (q2),
L¹ and L³ are each independently a single bond or a divalent spacer represented by any of the formula (z12) to the formula (z14) wherein s1 to s5 are each independently an integer of 0 to 10,
L², L⁴, L⁶, and L⁸ are each a single bond,
L⁹ is a divalent spacer represented by the formula (z15) or the formula (z16) wherein s6 is an integer of 1 to 10,
L⁵ and L⁷ are each independently a single bond or a divalent spacer represented by the formula (z17) wherein s7 is an integer of 1 to 10 and L¹⁰ is a degradable oligopeptide chain, and
R¹ and R² are each independently a hydrocarbon group having 1 to 5 carbon atoms.

In this embodiment, L¹⁰ is preferably a phenylalanine-glycine chain, and it is more preferred that L¹⁰ is a phenylalanine-glycine chain, an imino group (-NH-) of -(CH₂)ₛ₇-NH- and a carbonyl group (-CO-) of the glycine residue in L¹⁰ are bonded, and an imino group (-NH-) of the phenylalanine residue in L¹⁰ are bonded to Q¹ or Q².

In this embodiment, the explanation of the number average molecular weight of the divalent groups of the polyethylene glycol or polysialic acid for P¹ to P⁴ and the total thereof (preferred range, etc.) is the same as the explanation of the number average molecular weight of the divalent groups of the aforementioned water-soluble polymers for P¹ to P⁴ and the total thereof.

### [Water-soluble polymer (1-2) or water-soluble polymer (2-2)]

Water-soluble polymer (1) or water-soluble polymer (2), wherein
X¹ and X² are each independently an active ester group, a functional group represented by the formula (e) wherein Y¹ is a hydrogen atom or an alkyl group having 1 to 5 carbon atoms, a carboxy group, or an amino group,
P¹ to P⁴ are each independently a divalent group of polyethylene glycol,
Q¹ is a trivalent group represented by the formula (q1),
Q² is a trivalent group represented by the formula (q1) or the formula (q2),
L¹ and L³ are each independently a single bond or a divalent spacer represented by any of the formula (z12) to the formula (z14) wherein s1 to s5 are each independently an integer of 0 to 6,
L², L⁴, L⁶, and L⁸ are each a single bond,
L⁹ is a divalent spacer represented by the formula (z15) or the formula (z16) wherein s6 is an integer of 1 to 6,
L⁵ and L⁷ are each independently a single bond or a divalent spacer represented by the formula (z17) wherein each s7 is independently an integer of 1 to 6 and L¹⁰ is a degradable oligopeptide chain, and
R¹ and R² are each independently an alkyl group having 1 to 5 carbon atoms.

In this embodiment, L¹⁰ is preferably a phenylalanine-glycine chain, and it is more preferred that L¹⁰ is a phenylalanine-glycine chain, an imino group (-NH-) of -(CH₂)ₛ₇-NH- and a carbonyl group (-CO-) of the glycine residue in L¹⁰ are bonded, and an imino group (-NH-) of the phenylalanine residue in L¹⁰ are bonded to Q¹ or Q².

In this embodiment, the explanation of the number average molecular weight of the divalent groups of the polyethylene glycol for P¹ to P⁴ and the total thereof (preferred range, etc.) is the same as the explanation of the number average molecular weight of the divalent groups of the aforementioned water-soluble polymers for P¹ to P⁴ and the total thereof.

### [Water-soluble polymer (1-3) or water-soluble polymer (2-3)]

Water-soluble polymer (1) or water-soluble polymer (2), wherein
X¹ and X² are each independently a functional group represented by the formula (a) (i.e., N-succinimidyloxycarbonyl group), a maleimidyl group, a carboxy group, or an amino group,
P¹ to P⁴ are each independently a divalent group of polyethylene glycol,
Q¹ is a trivalent group represented by the formula (q1),
Q² is a trivalent group represented by the formula (q1) or the formula (q2),
L¹ and L³ are each independently a single bond or a divalent spacer represented by any of the formula (z12) to the formula (z14) wherein s1 to s5 are each independently an integer of 0 to 3, and s2 to s4 are each independently an integer of 1 to 3,
L², L⁴, L⁶, and L⁸ are each a single bond,
L⁹ is a divalent spacer represented by the formula (z15) or the formula (z16) wherein s6 is an integer of 1 to 5,
L⁵ and L⁷ are each independently a single bond or a divalent spacer represented by the formula (z17) wherein s7 is an integer of 1 to 3 and L¹⁰ is a degradable oligopeptide chain, and
R¹ and R² are each independently a methyl group or an ethyl group.

In this embodiment, L¹⁰ is preferably a phenylalanine-glycine chain, and it is more preferred that L¹⁰ is a phenylalanine-glycine chain, an imino group (-NH-) of -(CH₂)ₛ₇-NH- and a carbonyl group (-CO-) of the glycine residue in L¹⁰ are bonded, and an imino group (-NH-) of the phenylalanine residue in L¹⁰ are bonded to Q¹ or Q².

In this embodiment, the explanation of the number average molecular weight of the divalent groups of the polyethylene glycol for P¹ to P⁴ and the total thereof (preferred range, etc.) is the same as the explanation of the number average molecular weight of the divalent groups of the aforementioned water-soluble polymers for P¹ to P⁴ and the total thereof.

### [Water-soluble polymer (1-4) or water-soluble polymer (2-4)]

Water-soluble polymer (1) or water-soluble polymer (2), wherein
X¹ and X² are each independently a functional group represented by the formula (a) (i.e., N-succinimidyloxycarbonyl group), a maleimidyl group, a carboxy group, or an amino group,
P¹ to P⁴ are each independently a divalent group of polyethylene glycol,
Q¹ is a trivalent group represented by the formula (q1),
Q² is a trivalent group represented by the formula (q1) or the formula (q2),
L¹ and L³ are each independently a single bond or a divalent spacer represented by the formula (z13) wherein s3 is an integer of 1 to 3,
L², L⁴, L⁶, and L⁸ are each a single bond,
L⁹ is a divalent spacer represented by the formula (z15) or the formula (z16) wherein s6 is an integer of 1 to 5,
L⁵ and L⁷ are each independently a single bond or a divalent spacer represented by the formula (z17) wherein s7 is an integer of 1 to 3 and L¹⁰ is a degradable oligopeptide chain, and
R¹ and R² are each independently a methyl group or an ethyl group.

In this embodiment, L¹⁰ is preferably a phenylalanine-glycine chain, and it is more preferred that L¹⁰ is a phenylalanine-glycine chain, an imino group (-NH-) of -(CH₂)ₛ₇-NH- and a carbonyl group (-CO-) of the glycine residue in L¹⁰ are bonded, and an imino group (-NH-) of the phenylalanine residue in L¹⁰ are bonded to Q¹ or Q².

In this embodiment, the explanation of the number average molecular weight of the divalent groups of the polyethylene glycol for P¹ to P⁴ and the total thereof (preferred range, etc.) is the same as the explanation of the number average molecular weight of the divalent groups of the aforementioned water-soluble polymers for P¹ to P⁴ and the total thereof.

Preferred examples of water-soluble polymer (3) or water-soluble polymer (4) include the aforementioned [water-soluble polymer (1-1) or water-soluble polymer (2-1)] to [water-soluble polymer (1-4) or water-soluble polymer (2-4)], in which "X¹ and X²", "P¹ to P⁴", "L¹ and L³", "L², L⁴, L⁶, and L⁸", "L⁵ and L⁷", and "water-soluble polymer (1) or water-soluble polymer (2)" are respectively changed to "X¹", "P¹ and P³", "L¹", "L² and L⁶", "L⁵", and "water-soluble polymer (3) or water-soluble polymer (4)".

The water-soluble polymer of the present invention is particularly preferably compound (p3), compound (p4), compound (p4-a), compound (p6), compound (p7), compound (p7-a), compound (p9-a), compound (p15), or compound (p18), most preferably compound (p3), compound (p4), compound (p4-a), compound (p6), compound (p7), compound (p7-a), or compound (p9-a) of the below-mentioned Synthetic Examples.

The water-soluble polymer of the present invention can be produced as shown in the below-mentioned Synthetic Examples. For example, water-soluble polymer (1) can be produced by reacting polymer (1a) and polymer (1b) corresponding to the left and right structures of water-soluble polymer (1), as shown in the following formula: 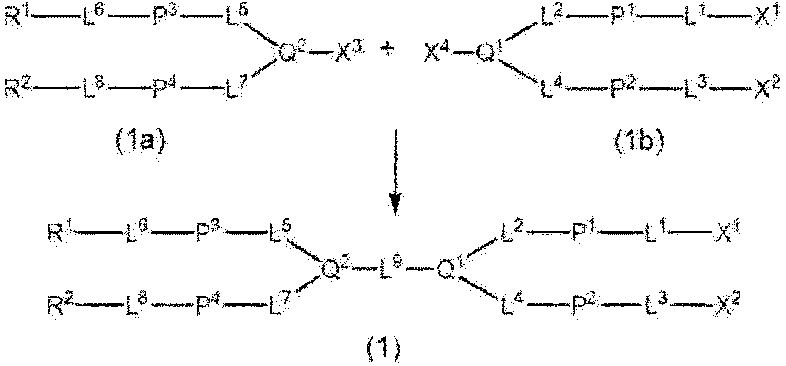 wherein X³ and X⁴ are functional groups or divalent spacers to which a functional group is bonded, and the functional group for X³ and the functional group for X⁴ are capable of reacting with each other, and other symbols are as defined above. Using a compound having a functional group capable of reacting with the functional group for X³ and a functional group capable of reacting with the functional group for X⁴, polymer (1a) and polymer (1b) may be bonded via the compound (i.e., spacer). Polymer (1a) and polymer (1b) may be commercially available products, or may be produced using known reactions, as shown in the below-mentioned Synthetic Examples. Polymer (1a) and polymer (1b) can be synthesized, for example, by binding water-soluble polymers corresponding to P¹ to P⁴ to glycerol or a derivative thereof, glutamic acid or a derivative thereof, or lysine or a derivative thereof, directly or via a spacer to form an intermediate, and binding a compound having X¹ or X² to the obtained intermediate directly or via a spacer.

### [Example]

The present invention is explained in more detail in the following by referring to Synthetic Examples and the like. In the following, "%" is "wt%" unless otherwise stated.

¹H-NMR obtained in the following Synthetic Examples was obtained from JNM-ECZ400 or JNM-ECA600 manufactured by JEOL Datam Co., Ltd. A ϕ5 mm tube was used for the measurement, and CDCl₃ and DMSO-d₆ containing tetramethylsilane (TMS) as an internal standard substance were used as deuterated solvents. The molecular weight of the obtained four-branched water-soluble polymers and the like was calculated using a gel permeation chromatography (GPC) system, Prominence manufactured by SHIMADZU CORPORATION. In addition, for confirmation of aggregation in Example, a high performance liquid chromatography (UHPLC) system, Ultimate 3000 manufactured by Thermo Fisher, was used. The analysis conditions of GPC and UHPLC are shown below.

### GPC analysis (molecular weight measurement)

detector: differential refractometer
column: PL gel MIXED-D (Agilent Technologies)
mobile phase: 10 mM LiBr+DMF
flow rate: 0.7 mL/min
sample amount: 1 mg/mL, 100 µL
column temperature: 65°C

### UHPLC analysis (confirmation of aggregation)

detector: UV
column: TSKgel UP-SW3000 (Tosoh Corporation)
mobile phase: 100 mM sodium phosphate buffer (pH 6.7)+100 mM Na₂SO₄+0.05% NaN₃
flow rate: 0.35 mL/min
injection volume: 5 mg/mL, 10 µL
column temperature: 25°C

### [Synthetic Example 1] Synthesis of compound (p1) represented by the following formula (in the following formula, n is about 114)

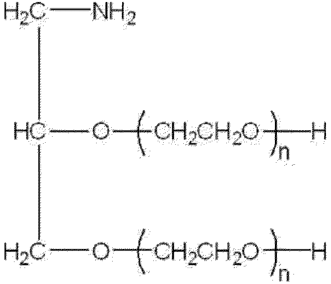

### [Synthetic Example 1-1] Synthesis of compound (p1-a) represented by the following formula (in the following formula, n is about 114)

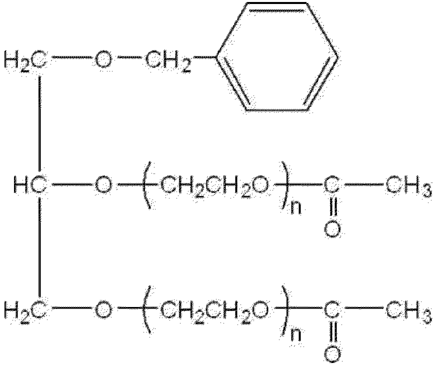

1-Benzyloxy-2,3-bis[hydro(polyoxyethylene)-oxy]propane (SUNBRIGHT GL2-100BH) manufactured by NOF CORPORATION (25 g, 2.5 mmol) was dissolved in toluene (75 g), followed by refluxing dehydrating at 110°C for 30 min. Thereafter, the solution was cooled to room temperature, dichloromethane (62.5 g), 4-dimethylaminopyridine (DMAP) (61.1 mg, 0.5 mmol), and triethylamine (TEA) (1.52 g, 15 mmol) were added, and the mixture was stirred at room temperature for 5 min. After stirring, to the obtained solution was added acetic anhydride (765 mg, 7.5 mmol), and the mixture was reacted at room temperature under a nitrogen atmosphere for 2 hr. After completion of the reaction, methanol (240 mg) was added, and the solution was stirred at room temperature under a nitrogen atmosphere for 30 min. Thereafter, the solution was concentrated to dryness, and the obtained concentrate was dissolved in toluene (150 g) at 40°C under a nitrogen atmosphere. The obtained solution was cooled to room temperature, hexane (75 g) was added, and the mixture was stirred at room temperature for 15 min and the resultant product was precipitated. Suction filtration was performed using filter paper 5A, the precipitate was collected, dissolved again in toluene (150 g), hexane (75 g) was added, and the mixture was stirred at room temperature for 15 min and the resultant product was precipitated. Suction filtration was performed using filter paper 5A, and the precipitate was collected and washed with hexane (100 g). Suction filtration was performed using filter paper 5A, and vacuum drying gave the above-mentioned compound (p1-a) (yield 21.5 g).

### [Synthetic Example 1-2] Synthesis of compound (p1-b) represented by the following formula (in the following formula, n is about 114)

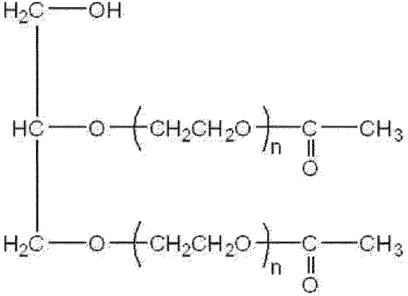

The compound (p1-a) (21 g, 2.1 mmol) obtained in Synthetic Example 1-1 and palladium/carbon (manufactured by N.E. CHEMCAT CORPORATION, 10.5 g) were charged in a reactor, methanol (210 mL) cooled to 5°C or below and cyclohexene (35.1 g) were added, and the mixture was heated to 45°C and reacted for 6 hr under a nitrogen atmosphere. After completion of the reaction, palladium/carbon was removed by filtration through filter paper 5A. The filtrate was concentrated to dryness, the obtained concentrate was dissolved in toluene (105 g) at 40°C, the obtained solution was cooled to room temperature, hexane (63 g) was added, and the resultant product was precipitated. Suction filtration was performed using filter paper 5A, and the precipitate was collected and washed with hexane (84 g). Suction filtration was performed using filter paper 5A, and vacuum drying gave the above-mentioned compound (p1-b) (yield 19 g).

### [Synthetic Example 1-3] Synthesis of compound (p1)

The compound (p1-b) (18 g, 1.8 mmol) obtained in Synthetic Example 1-2 was dissolved at 40°C in toluene (144 g) containing 2,6-di-tert-butyl-p-cresol (hereinafter referred to as "BHT") (18 mg), the mixture was heated to 110°C, and refluxing dehydrating was performed for 30 min. Thereafter, the solution was cooled to room temperature, dehydrated dichloromethane (18 g) was added, and the mixture was stirred to uniformity. Phthalimide (794 mg, 5.4 mmol), triphenylphosphine (1.42 g, 5.4 mmol), and diisopropyl azodicarboxylate (1.09 g, 5.4 mmol) were added, and the mixture was reacted at room temperature under a nitrogen atmosphere for 2 hr. After completion of the reaction, methanol (173 mg, 5.4 mmol) was added, and the solution was stirred for 1 hr. Thereafter, the solution was concentrated to 70 g, methanol (54 g) was added to the obtained concentrate, and the mixture was stirred at room temperature under a nitrogen atmosphere to uniformity. After stirring, to the obtained solution was added ethylenediamine monohydrate (14.1 g, 180 mmol), and the mixture was reacted at 40°C under a nitrogen atmosphere for 5 hr. After completion of the reaction, the solution was cooled to room temperature, 20% brine (112.5 g) was added, and the mixture was stirred for 10 min. Thereafter, dichloromethane (180 g) was added, the solution was stirred at room temperature under a nitrogen atmosphere for 30 min. After stirring, the solution was allowed to stand for 30 min for partitioning, and the organic layer was recovered. The recovered organic layer was concentrated to dryness, and the obtained concentrate was dissolved in ethyl acetate (180 g). To the obtained solution was added sodium sulfate (18 g), and the mixture was stirred at room temperature under a nitrogen atmosphere for 30 min for dehydration. After dehydrating, sodium sulfate was removed by suction filtration through filter paper 5A. To the obtained filtrate was added hexane (90 g), the mixture was stirred at room temperature for 15 min, and the resultant product was precipitated. Suction filtration was performed using filter paper 5A, and the precipitate was collected and washed with hexane (72 g). Suction filtration was performed using filter paper 5A, and vacuum drying gave the above-mentioned compound (p1) (yield 15.2 g).

### [Synthetic Example 2] Synthesis of compound (p2) represented by the following formula (in the following formula, m is about 228 and n is about 114)

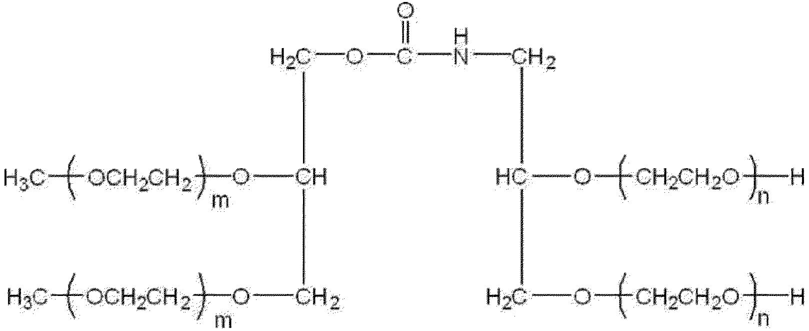

The compound (p1) (3.0 g, 0.3 mmol) obtained in Synthetic Example 1-3 and SUNBRIGHT GL2-200TS manufactured by NOF CORPORATION (6.0 g, 0.3 mmol) were dissolved in toluene (36 g), and the mixture was reacted at 40°C under a nitrogen atmosphere for 3 hr. After completion of the reaction, the solution was diluted with toluene (108 g) and stirred at room temperature for 5 min. Thereafter, hexane (60 g) was added, and the mixture was stirred at room temperature under a nitrogen atmosphere for 15 min, and the resultant product was precipitated. Suction filtration was performed using filter paper 5A, and the precipitate was collected and washed with hexane (36 g). Suction filtration was performed using filter paper 5A, and vacuum drying gave the above-mentioned compound (p2) (yield 7.8 g).

### [Synthetic Example 3] Synthesis of compound (p3) represented by the following formula (in the following formula, m is about 228 and n is about 114)

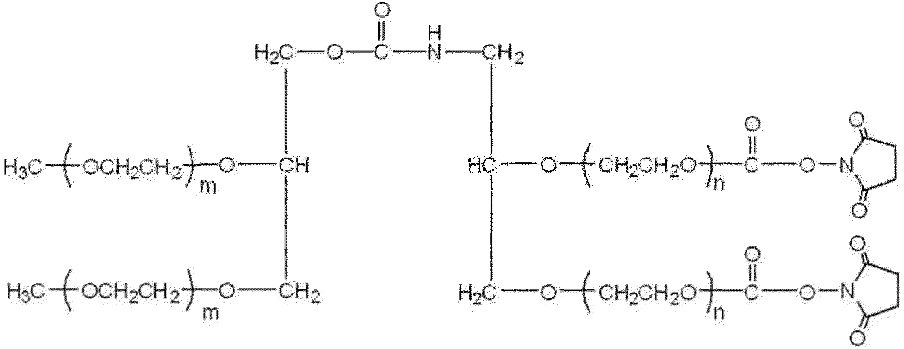

The compound (p2) (3.0 g, 0.1 mmol) obtained in Synthetic Example 2 was dissolved in dichloromethane (10.5 g), pyridine (119 mg, 1.5 mmol) and di(N-succinimidyl)carbonate (256 mg, 1.0 mmol) were added and the mixture was reacted at room temperature under a nitrogen atmosphere for 15 hr. After completion of the reaction, the reaction solution was concentrated to dryness. The obtained concentrate was dissolved in ethyl acetate (100 g) at 40°C under a nitrogen atmosphere, and the mixture was allowed to cool to room temperature. Hexane (50 g) was added, and the mixture was stirred at room temperature under a nitrogen atmosphere for 15 min, and the resultant product was precipitated. Suction filtration was performed using filter paper 5A, the precipitate was collected and dissolved again in ethyl acetate (100 g). Hexane (50 g) was added and the resultant product was precipitated. Suction filtration was performed using filter paper 5A, and the precipitate was collected and washed with hexane (50 g). Suction filtration was performed using filter paper 5A, and vacuum drying gave the above-mentioned compound (p3) (yield 2.3 g, number average molecular weight (Mn) 34,510).

### [Synthetic Example 4] Synthesis of compound (p4) represented by the following formula (in the following formula, m is about 228 and n is about 114)

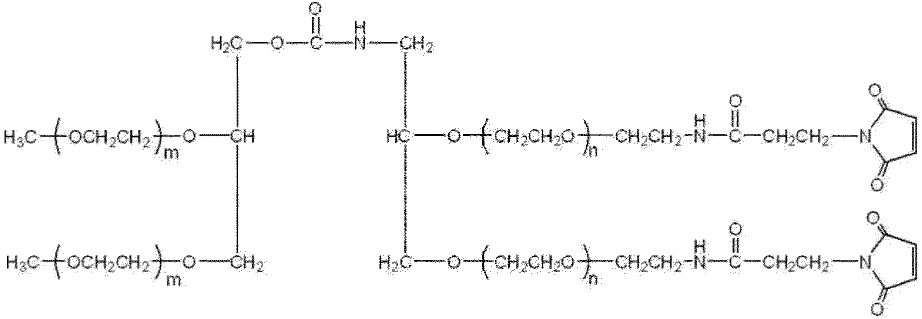

### [Synthetic Example 4-1] Synthesis of compound (p4-a) represented by the following formula (in the following formula, m is about 228 and n is about 114)

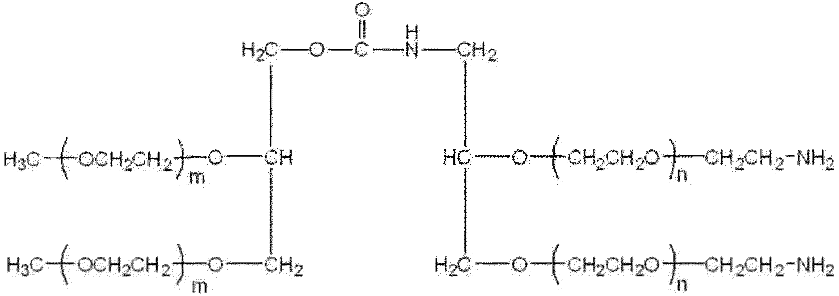

The compound (p2) (3.0 g, 0.1 mmol) obtained in Synthetic Example 2 was dissolved at 40°C in toluene (24 g) containing BHT (30 mg), the mixture was heated to 110°C and refluxing dehydrating was performed for 30 min. Thereafter, the solution was cooled to room temperature, dehydrated dichloromethane (3.0 g) was added, and the mixture was stirred to uniformity. Phthalimide (88 mg, 0.6 mmol), triphenylphosphine (157 mg, 0.6 mmol), and diisopropyl azodicarboxylate (121 mg, 0.6 mmol) were added, and the mixture was reacted at room temperature under a nitrogen atmosphere for 2 hr. After completion of the reaction, methanol (20 mg) was added and the solution was stirred for 1 hr. Thereafter, the solution was concentrated to 12 g, methanol (30 g) was added to the obtained concentrate, and the mixture was stirred at room temperature under a nitrogen atmosphere to uniformity. After stirring, to the obtained solution was added ethylenediamine monohydrate (780 mg, 10 mmol) and the mixture was reacted at 40°C under a nitrogen atmosphere for 5 hr. After completion of the reaction, the solution was cooled to room temperature, 20% brine (18.75 g) was added, and the solution was stirred for 10 min. Thereafter, dichloromethane (30 g) was added, and the solution was stirred at room temperature under a nitrogen atmosphere for 30 min. After stirring, the solution was allowed to stand for 30 min for partitioning, and the organic layer was recovered. The recovered organic layer was concentrated to dryness, and the obtained concentrate was dissolved in ethyl acetate (100 g). To the obtained solution was added sodium sulfate (3 g) and the mixture was stirred at room temperature under a nitrogen atmosphere for 30 min to perform a dehydration operation. After dehydration, sodium sulfate was removed by suction filtration through filter paper 5A. To the obtained filtrate was added hexane (50 g) and the mixture was stirred at temperature for 15 min and the resultant product was precipitated. Suction filtration was performed using filter paper 5A, and the precipitate was collected and washed with hexane (50 g). Suction filtration was performed using filter paper 5A, and vacuum drying gave the above-mentioned compound (p4-a) (yield 2.3 g) .

### [Synthetic Example 4-2] Synthesis of compound (p4)

The compound (p4-a) (2.1 g, 0.07 mmol) obtained in Synthetic Example 4-1 was dissolved in a mixed solvent of toluene (10.9 g) and acetonitrile (1.7 g), N-methylmorpholine (71 mg, 0.7 mmol) was added, and the mixture was stirred to uniformity under a nitrogen atmosphere. After stirring, to the obtained solution was added N-succinimidyl 3-maleimidopropionate (186 mg, 0.7 mmol) and the mixture was reacted at room temperature under a nitrogen atmosphere for 4 hr. After completion of the reaction, the mixture was diluted with ethyl acetate (100 g) and stirred to uniformity at room temperature under a nitrogen atmosphere. Thereafter, hexane (50 g) was added, and the mixture was stirred at room temperature for 30 min, and the resultant product was precipitated. Suction filtration was performed using filter paper 5A, and the precipitate was collected and dissolved in ethyl acetate (100 g). Hexane (50 g) was added and the mixture was stirred at room temperature for 30 min and the resultant product was precipitated. Suction filtration was performed using filter paper 5A, and the precipitate was collected and washed with hexane (50 g). Suction filtration was performed using filter paper 5A, and vacuum drying gave the above-mentioned compound (p4) (yield 1.5 g, number average molecular weight (Mn) 33,420).

### [Synthetic Example 5] Synthesis of compound (p5) represented by the following formula (in the following formula, m is about 228 and n is about 114)

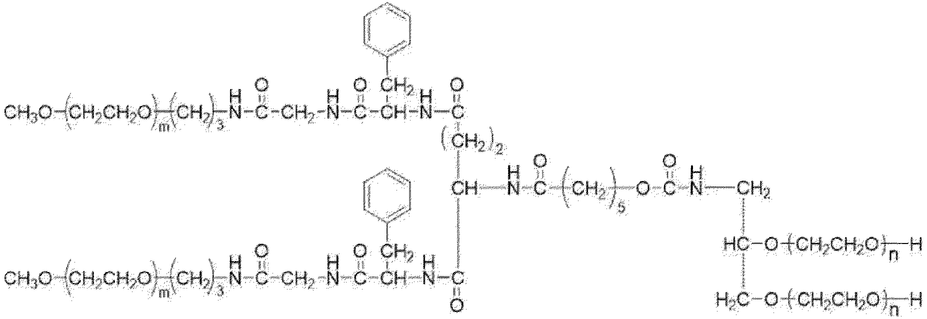

The compound (p1) (3.0 g, 0.15 mmol) obtained in Synthetic Example 1-3 and SUNBRIGHT BP2-200TS2 manufactured by NOF CORPORATION (6.0 g, 0.3 mmol) were dissolved in toluene (36 g), and the mixture was reacted at 40°C under a nitrogen atmosphere for 3 hr. After completion of the reaction, the solution was diluted with toluene (108 g), and the mixture was stirred at room temperature for 5 min. Thereafter, hexane (60 g) was added, and the mixture was stirred at room temperature under a nitrogen atmosphere for 15 min and the resultant product was precipitated. Suction filtration was performed using filter paper 5A, and the precipitate was collected and washed with hexane (36 g). Suction filtration was performed using filter paper 5A, and vacuum drying gave the above-mentioned compound (p5) (yield 7.4 g).

### [Synthetic Example 6] Synthesis of compound (p6) represented by the following formula (in the following formula, m is about 228 and n is about 114)

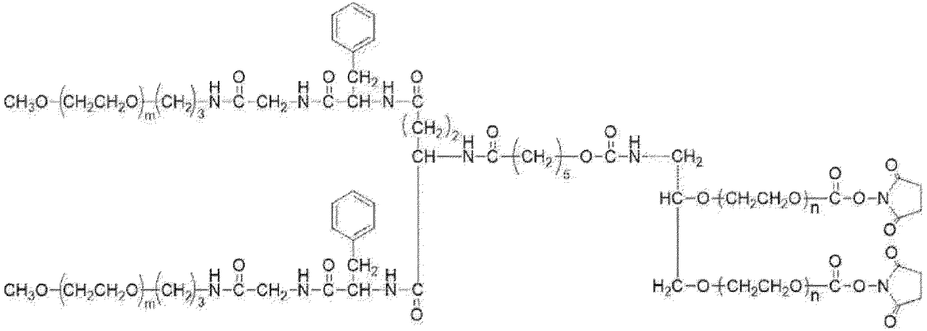

The compound (p5) (3.0 g, 0.1 mmol) obtained in Synthetic Example 5 was dissolved in dichloromethane (10.5 g), pyridine (119 mg, 1.5 mmol) and di(N-succinimidyl)carbonate (256 mg, 1.0 mmol) were added and the mixture was reacted at room temperature under a nitrogen atmosphere for 15 hr. After completion of the reaction, the reaction solution was concentrated to dryness. The obtained concentrate was dissolved in ethyl acetate (100 g) at 40°C under a nitrogen atmosphere and allowed to cool to room temperature. Hexane (50 g) was added, the mixture was stirred at room temperature under a nitrogen atmosphere for 15 min, and the resultant product was precipitated. Suction filtration was performed using filter paper 5A, the precipitate was collected and dissolved again in ethyl acetate (100 g). Hexane (50 g) was added and the resultant product was precipitated. Suction filtration was performed using filter paper 5A, and the precipitate was collected and washed with hexane (50 g). Suction filtration was performed using filter paper 5A, and vacuum drying gave the above-mentioned compound (p6) (yield 2.4 g, number average molecular weight (Mn) 35,780).

### [Synthetic Example 7] Synthesis of compound (p7) represented by the following formula (in the following formula, m is about 228 and n is about 114)

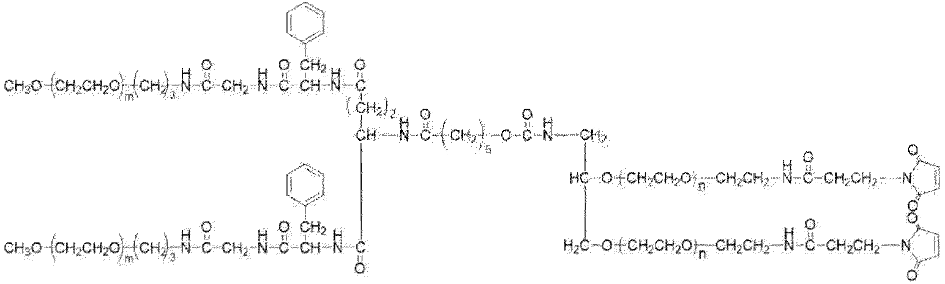

### [Synthetic Example 7-1] Synthesis of compound (p7-a) represented by the following formula (in the following formula, m is about 228 and n is about 114)

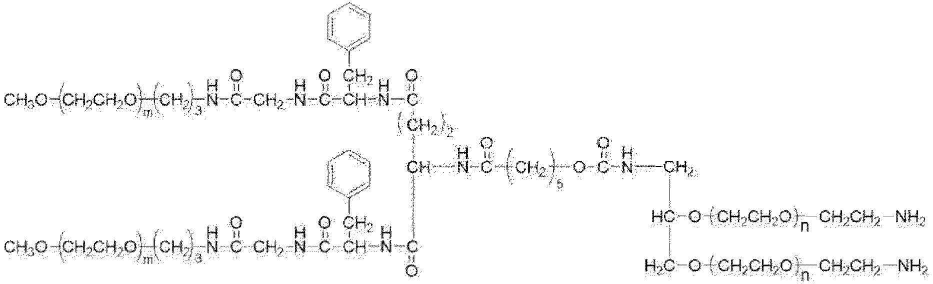

The compound (p5) (3.0 g, 0.1 mmol) obtained in Synthetic Example 5 was dissolved at 40°C in toluene (24 g) containing BHT (30 mg), the mixture was heated to 110°C, and refluxing dehydrating was performed for 30 min. Thereafter, the solution was cooled to room temperature, dehydrated dichloromethane (3.0 g) was added, and the mixture was stirred to uniformity. Phthalimide (88 mg, 0.6 mmol), triphenylphosphine (157 mg, 0.6 mmol), and diisopropyl azodicarboxylate (121 mg, 0.6 mmol) were added and the mixture was reacted at room temperature under a nitrogen atmosphere for 2 hr. After completion of the reaction, methanol (20 mg) was added, and the solution was stirred for 1 hr. Thereafter, the solution was concentrated to 12 g, methanol (30 g) was added to the obtained concentrate, and the mixture was stirred to uniformity at room temperature under a nitrogen atmosphere. After stirring, to the obtained solution was added ethylenediamine monohydrate (780 mg, 10 mmol), and the mixture was reacted at 40°C under a nitrogen atmosphere for 5 hr. After completion of the reaction, the solution was cooled to room temperature, 20% brine (18.75 g) was added, and the mixture was stirred for 10 min. Thereafter, dichloromethane (30 g) was added, and the solution was stirred at room temperature under a nitrogen atmosphere for 30 min. After stirring, the solution was allowed to stand for 30 min for partitioning, and the organic layer was recovered. The recovered organic layer was concentrated to dryness, and the obtained concentrate was dissolved in ethyl acetate (100 g). To the obtained solution was added sodium sulfate (3 g), and the mixture was stirred at room temperature under a nitrogen atmosphere for 30 min to perform a dehydration operation. After dehydration, sodium sulfate was removed by suction filtration through filter paper 5A. To the obtained filtrate was added hexane (50 g) and the mixture was stirred at room temperature for 15 min and the resultant product was precipitated. Suction filtration was performed using filter paper 5A, and the precipitate was collected and washed with hexane (50 g). Suction filtration was performed using filter paper 5A, and vacuum drying gave the above-mentioned compound (p7-a) (yield 2.1 g).

### [Synthetic Example 7-2] Synthesis of compound (p7)

The compound (p7-a) (2.1 g, 0.07 mmol) obtained in Synthetic Example 7-1 was dissolved in a mixed solvent of toluene (10.9 g) and acetonitrile (1.7 g), N-methylmorpholine (71 mg, 0.7 mmol) was added, and the mixture was stirred to uniformity under a nitrogen atmosphere. After stirring, to the obtained solution was added N-succinimidyl 3-maleimidopropionate (186 mg, 0.7 mmol) and the mixture was reacted at room temperature under a nitrogen atmosphere for 4 hr. After completion of the reaction, the mixture was diluted with ethyl acetate (100 g) and stirred to uniformity at room temperature under a nitrogen atmosphere. Thereafter, hexane (50 g) was added, the mixture was stirred at room temperature for 30 min, and the resultant product was precipitated. Suction filtration was performed using filter paper 5A, and the precipitate was collected and dissolved again in ethyl acetate (100 g). Hexane (50 g) was added, the mixture was stirred at room temperature for 30 min, and the resultant product was precipitated. Suction filtration was performed using filter paper 5A, and the precipitate was collected and washed with hexane (50 g). Suction filtration was performed using filter paper 5A, and vacuum drying gave the above-mentioned compound (p7) (yield 1.5 g, number average molecular weight (Mn) 36,240).

### [Synthetic Example 8] Synthesis of compound (p8) represented by the following formula (in the following formula, m is about 228, n is about 114, and -S-_{Trast}Fab' is a group having a structure obtained by removing the hydrogen atom of the mercapto group from trastuzumab fragment (HS-_{Trast}Fab'))

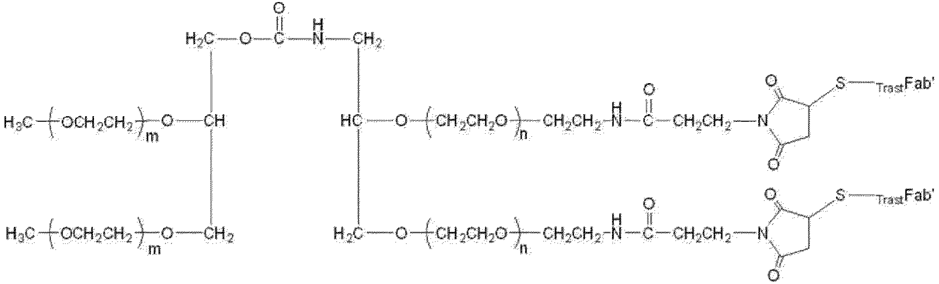

### [Synthetic Example 8-1] Preparation of _{Trast}F(ab')₂ solution

Trastuzumab manufactured by Selleck Biotech, Co., Ltd. (8 mg) and pepsin (0.4 mg) (Sigma Aldrich) were dissolved in a 10 mM EDTA-containing 100 mM acetate buffer (2 mL) at pH 4.0, and the mixture was reacted at 37°C for 16 hr. After the reaction, the reaction solvent was exchanged with 20 mM acetate buffer at pH 4.5 by dialysis, and purified by cation exchange column chromatography to recover a fraction solution containing _{Trast}F(ab')₂. The recovered fraction solution was concentrated to 1 mL by ultrafiltration, and the obtained concentrate was diluted with a 100 mM phosphate buffer (20 mL) at pH 6.0, and concentrated again to 1 mL by ultrafiltration. A similar operation was repeated two more times, the absorbance at 280 nm was measured using Nanodrop (Thermo Scientific), and the protein concentration was confirmed. After confirmation, ultrafiltration was performed, and 4 mg/mL _{Trast}F(ab')₂ solution (0.8 mL) was prepared by concentration.

### [Synthetic Example 8-2] Preparation of _{Trast}Fab' solution

To the 4 mg/mL _{Trast}F(ab')₂ solution (0.5 mL) prepared in Synthetic Example 8-1 was added a 4 mM EDTA-containing 100 mM phosphate buffer (0.5 mL) at pH 6.0 containing 10 mM cysteamine, and the mixture was reacted at room temperature for 16 hr. After the reaction, the reaction solution was passed through a gel filtration column equilibrated in advance with a 10 mM EDTA-containing 20 mM acetate buffer at pH 4.5, and a fraction solution containing _{Trast}Fab' was recovered. The recovered solution was concentrated to 1 mL by ultrafiltration, and the obtained concentrate was diluted with a 20 mM EDTA-containing 100 mM phosphate buffer (20 mL) at pH 6.0, and concentrated again to 1 mL by ultrafiltration. A similar operation was repeated two more times, the absorbance at 280 nm was measured using Nanodrop (Thermo Scientific), and the protein concentration was confirmed. After confirmation, 4 mg/mL _{Trast}Fab' solution (0.2 mL) was prepared by concentration by ultrafiltration.

### [Synthetic Example 8-3] Synthesis of compound (p8)

To 4 mg/ml _{Trast}Fab' solution (0.1 mL) obtained in Synthetic Example 8-2 was added 2 equal amounts of the compound (p4) obtained in Synthetic Example 4-2 and dissolved in 20 mM EDTA-containing 100 mM phosphate buffer at pH 6.0 such that the total solution amount was 0.2 mL, and the mixture was reacted at room temperature for 6 hr. After the reaction, a fraction solution containing the compound (p8) was recovered by ion exchange chromatography and gel filtration column chromatography. The recovered fraction solution was concentrated by ultrafiltration, and the obtained concentrate was diluted with 5% sucrose-containing PBS and concentrated again to 1 mL by ultrafiltration. A similar operation was repeated one more time, and the obtained concentrate was freeze-dried to give a compound (p8) solution (yield 0.3 mg).

### [Synthetic Example 9] Synthesis of compound (p9) represented by the following formula (in the following formula, m is about 228, n is about 114, and -O-Ciclosporin is a group having a structure obtained by removing the hydrogen atom of the hydroxy group from ciclosporin A (HO-Ciclosporin))

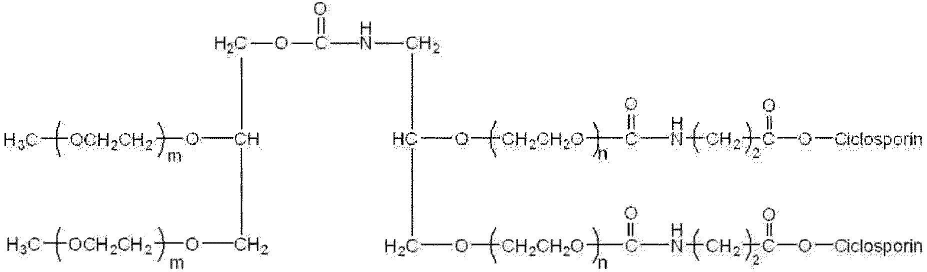

### [Synthetic Example 9-1] Synthesis of compound (p9-a) represented by the following formula (in the following formula, m is about 228 and n is about 114)

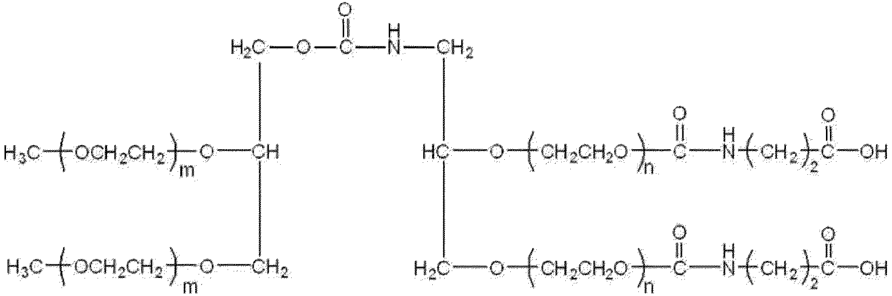

The compound (p3) (150 mg, 0.005 mmol) obtained in Synthetic Example 3 and 3-aminopropanoic acid (4.5 mg, 0.05 mmol) were dissolved in a 100 mM sodium borate buffer (0.5 mL) at pH 8.5, and the mixture was reacted at room temperature under a nitrogen atmosphere for 5 hr. After completion of the reaction, the solution was diluted with 20% brine (5 mL), chloroform (5 mL) was added, and the obtained mixture was stirred at room temperature for 10 min. After stirring, the mixture was allowed to stand for 10 min for partitioning, and the organic layer was recovered. To the recovered organic layer was added magnesium sulfate (100 mg) and the mixture was stirred at room temperature for 10 min to perform a dehydration operation. After dehydration, magnesium sulfate was removed by suction filtration through filter paper 5A. The obtained filtrate was diluted with toluene (100 mL), and the mixture was stirred to uniformity. After stirring, hexane (100 mL) was added to the solution, the mixture was stirred at room temperature under a nitrogen atmosphere for 15 min, and the resultant product was precipitated. Suction filtration was performed using filter paper 5A, and the precipitate was collected and washed with hexane (50 mL). Suction filtration was performed using filter paper 5A, and vacuum drying gave the above-mentioned compound (p10-a) (yield 110 mg).

### [Synthetic Example 9-2] Synthesis of compound (p9)

The compound (p9-a) (90 mg, 0.003 mmol) obtained in Synthetic Example 10-1 and ciclosporin A manufactured by FUJIFILM Wako Pure Chemical Corporation (36 mg, 0.03 mmol) were dissolved in dichloromethane (0.5 mL). To the solution was added dicyclohexylcarbodiimide (6 mg, 0.03 mmol) and the mixture was reacted at room temperature under a nitrogen atmosphere for 6 hr. After completion of the reaction, the reaction solution was diluted with toluene (100 mL). Suction filtration was performed using filter paper 5A, and insoluble material was removed by filtration. To the filtrate was added hexane (100 mL), the mixture was stirred at room temperature under a nitrogen atmosphere for 15 min, and the resultant product was precipitated. Suction filtration was performed using filter paper 5A, and the precipitate was collected and washed with hexane (50 mL). Suction filtration was performed using filter paper 5A, and vacuum drying gave the above-mentioned compound (p9) (50 mg).

### [Synthetic Example 10] Synthesis of compound (p10) represented by the following formula (in the following formula, m is about 228, n is about 114, and -NH-Linaclotide is a group having a structure obtained by removing the hydrogen atom of the amino group from Linaclotide (H₂N-Linaclotide))

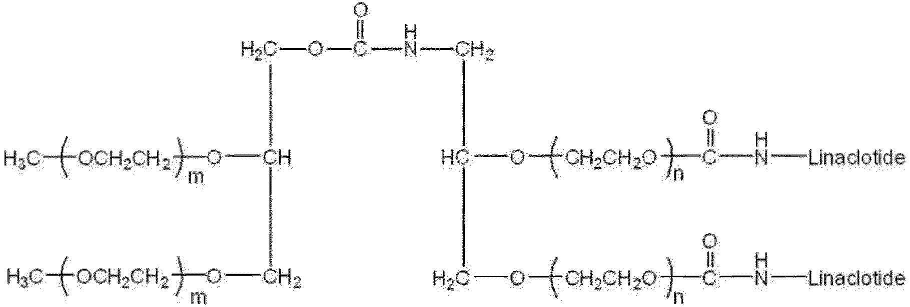

The compound (p3) (30 mg, 0.001 mmol) obtained in Synthetic Example 3 and Linaclotide manufactured by Medchemexpress (7.6 mg, 0.005 mmol) were dissolved in dimethyl sulfoxide (0.5 mL), and the mixture was reacted at room temperature under a nitrogen atmosphere for 6 hr. After completion of the reaction, the solution was diluted with toluene (100 mL) and stirred to uniformity. After stirring, suction filtration was performed using filter paper 5A. To the obtained filtrate was added hexane (100 mL), the mixture was stirred at room temperature under a nitrogen atmosphere for 15 min, and the resultant product was precipitated. Suction filtration was performed using filter paper 5A, and the precipitate was collected and washed with hexane (50 mL). Suction filtration was performed using filter paper 5A, and vacuum drying gave the above-mentioned compound (p10) (yield 20 mg) .

### [Synthetic Example 11] Synthesis of compound (p11) represented by the following formula (in the following formula, n is about 114, and -O-Ciclosporin is a group having a structure obtained by removing the hydrogen atom of the hydroxy group from ciclosporin A (HO-Ciclosporin))

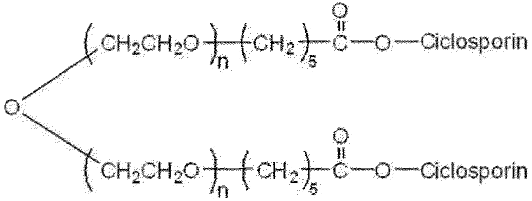

SUNBRIGHT DE-200HC manufactured by NOF CORPORATION (100 mg, 0.005 mmol) and ciclosporin A manufactured by FUJIFILM Wako Pure Chemical Corporation (60 mg, 0.05 mmol) were dissolved in dichloromethane (0.5 mL). To the solution was added dicyclohexylcarbodiimide (10 mg, 0.05 mmol) and the mixture was reacted at room temperature under a nitrogen atmosphere for 6 hr. After completion of the reaction, the mixture was diluted with toluene (100 mL). Suction filtration was performed using filter paper 5A, and the insoluble material was removed by filtration. To the filtrate was added hexane (100 mL), the mixture was stirred at room temperature under a nitrogen atmosphere for 15 min, and the resultant product was precipitated. Suction filtration was performed using filter paper 5A, and the precipitate was collected and washed with hexane (50 mL). Suction filtration was performed using filter paper 5A, and vacuum drying gave the above-mentioned compound (p11) (yield 65 mg).

### [Synthetic Example 12] Synthesis of compound (p12) represented by the following formula (in the following formula, n is about 114, and -NH-Linaclotide is a group having a structure obtained by removing the hydrogen atom of the amino group from Linaclotide (H₂N-Linaclotide))

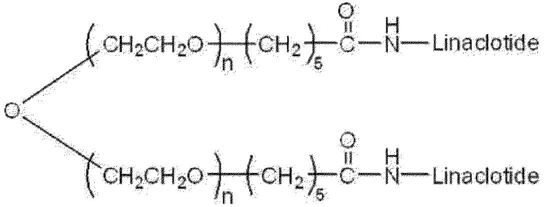

SUNBRIGHT DE-200HS manufactured by NOF CORPORATION (30 mg, 0.0015 mmol) and Linaclotide manufactured by Medchemexpress (11.4 mg, 0.0075 mmol) were dissolved in dimethyl sulfoxide (0.5 mL), and the mixture was reacted at room temperature under a nitrogen atmosphere for 6 hr. After completion of the reaction, the solution was diluted with toluene (100 mL) and stirred to uniformity. After stirring, suction filtration was performed using filter paper 5A, and hexane (100 mL) was added to the obtained filtrate. The mixture was stirred at room temperature under a nitrogen atmosphere for 15 min and the resultant product was precipitated. Suction filtration was performed using filter paper 5A, and the precipitate was collected and washed with hexane (50 mL). Suction filtration was performed using filter paper 5A, and vacuum drying gave the above-mentioned compound (p12) (yield 23 mg) .

### [Synthetic Example 13] Synthesis of compound (p13) represented by the following formula (in the following formula, n is about 227, and -NH-Linaclotide is a group having a structure obtained by removing the hydrogen atom of the amino group from Linaclotide (H₂N-Linaclotide))

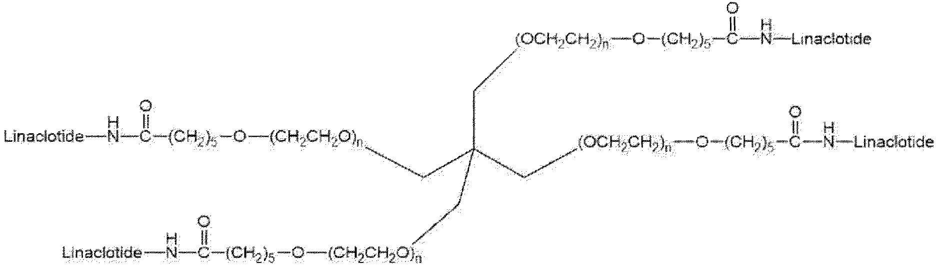

In the same manner as in Synthetic Example 10 except that SUNBRIGHT PTE-400HS manufactured by NOF CORPORATION (40 mg, 0.001 mmol) and Linaclotide manufactured by Medchemexpress (9.2 mg, 0.006 mmol) were used instead of the compound (p3) (30 mg, 0.001 mmol) obtained in Synthetic Example 3 and Linaclotide manufactured by Medchemexpress (7.6 mg, 0.005 mmol), the above-mentioned compound (p13) was obtained (yield 23 mg).

### [Synthetic Example 14] Synthesis of compound (p14) represented by the following formula (in the following formula, n is about 455)

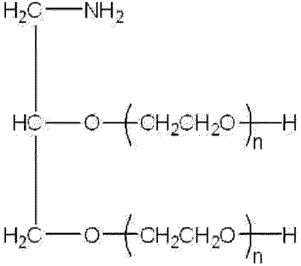

In the same manner as in Synthetic Example 1 except that 1-benzyloxy-2,3-bis[hydro(polyoxyethylene)-oxy]propane (SUNBRIGHT GL2-400BH) manufactured by NOF CORPORATION (30 g, 0.75 mmol) was used instead of 1-benzyloxy-2,3-bis[hydro(polyoxyethylene)-oxy]propane (SUNBRIGHT GL2-100BH) manufactured by NOF CORPORATION (25 g, 2.5 mmol), the above-mentioned compound (p14) was obtained (yield 8.2 g).

### [Synthetic Example 15] Synthesis of compound (p15) represented by the following formula (in the following formula, m and n are each about 455)

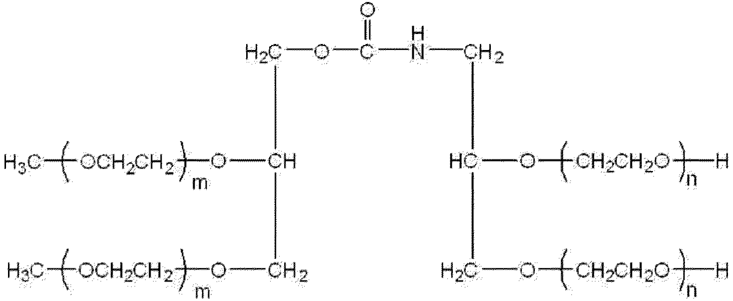

In the same manner as in Synthetic Example 2 except that the compound (p14) (4.0 g, 0.1 mmol) obtained in Synthetic Example 14 and SUNBRIGHT GL2-400TS manufactured by NOF CORPORATION (4.0 g, 0.1 mmol) were used instead of the compound (p1) (3.0 g, 0.3 mmol) obtained in Synthetic Example 1-3 and SUNBRIGHT GL2-200TS manufactured by NOF CORPORATION (6.0 g, 0.3 mmol), the above-mentioned compound (p15) was obtained (yield 6.8 g).

### [Synthetic Example 16] Synthesis of compound (p16) represented by the following formula (in the following formula, m and n are each about 455)

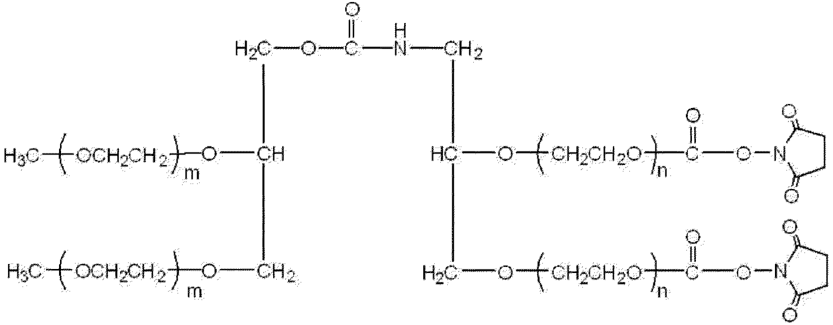

In the same manner as in Synthetic Example 3 except that the compound (p15) (4.0 g, 0.05 mmol) obtained in Synthetic Example 15 was used instead of the compound (p2) (3.0 g, 0.1 mmol) obtained in Synthetic Example 2, the above-mentioned compound (p16) was obtained (yield 2.8 g, number average molecular weight (Mn) 76,920).

### [Synthetic Example 17] Synthesis of compound (p17) represented by the following formula (in the following formula, m and n are each about 114)

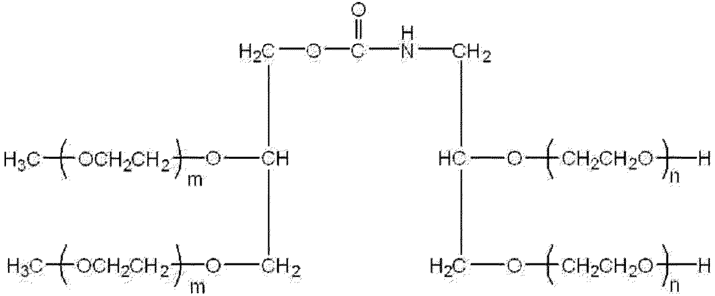

In the same manner as in Synthetic Example 2 except that SUNBRIGHT GL2-100TS manufactured by NOF CORPORATION (3.0 g, 0.3 mmol) was used instead of SUNBRIGHT GL2-200TS manufactured by NOF CORPORATION (6.0 g, 0.3 mmol), the above-mentioned compound (p17) was obtained (yield 4.1 g).

### [Synthetic Example 18] Synthesis of compound (p18) represented by the following formula (in the following formula, m and n are each about 114)

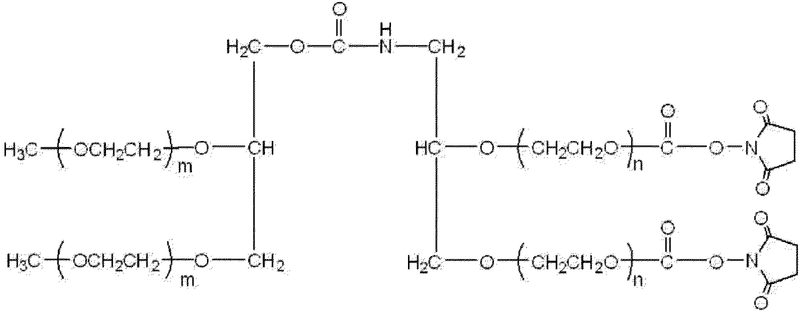

In the same manner as in Synthetic Example 3 except that the compound (p17) (3.0 g, 0.15 mmol) obtained in Synthetic Example 17 was used instead of the compound (p2) (3.0 g, 0.1 mmol) obtained in Synthetic Example 2, the above-mentioned compound (p18) was obtained (yield 1.3 g, number average molecular weight (Mn) 21, 120) .

### [Experimental Example 1]

Aggregation of the compound (p8) obtained in Synthetic Example 8 and trastuzumab in an aqueous solution was confirmed for each. First, the compound (p8) was dissolved in ultrapure water and 1 M hydrochloric acid was added to adjust to pH 1.0. Thereafter, 1 M sodium hydroxide aqueous solution was added to adjust to pH 10.0, after which 1 M hydrochloric acid was added again to adjust to pH 6.0 to give an aqueous solution of compound (p8). Then, trastuzumab was dissolved in PBS containing 5% sucrose, and a similar operation as above was performed to give a trastuzumab aqueous solution at pH 6.0. The obtained respective aqueous solutions were maintained at 60°C for 1 hr and UHPLC analysis was performed. As a result, an aggregate peak was confirmed at a retention time shorter than the main peak in the trastuzumab aqueous solution, but no aggregate peak was found in the aqueous solution of compound (p8), which confirms the aggregation suppressive effect of compound (p8).

### [Experimental Example 2]

The solubility when 10 mg each of the compound (p9) obtained in Synthetic Example 9, the compound (p11) obtained in Synthetic Example 11, and cyclosporin A was added to 1.0 mL of PBS and stirred at 25°C for 15 min was confirmed. As a result, the compound (p9) was dissolved, and the compound (p11) was slightly suspended. In addition, cyclosporin A was hardly dissolved, which confirmed the solubility improving effect of compound (p9).

### [Experimental Example 3]

The solubility of the compound (p10) obtained in Synthetic Example 10, the compound (p12) obtained in Synthetic Example 12, the compound (p13) obtained in Synthetic Example 13, and Linaclotide was confirmed from the time required for dissolution when 10 mg each thereof was added to 1.0 mL of PBS and stirred at 25°C. As a result, compound (p10) was completely dissolved after stirring for 2 min. On the other hand, compound (p12), compound (p13), and Linaclotide did not dissolve even after stirring for 15 min. Therefrom the solubility improving effect of compound (p10) was confirmed.

### [Industrial Applicability]

The four-branched water-soluble polymer for medical use of the present invention can improve solubility of conjugates thereof with biologically relevant materials and can suppress aggregation of the aforementioned conjugates. Furthermore, since the four-branched water-soluble polymer for medical use of the present invention has two functional groups capable of reacting with a biologically relevant material in one molecule, a conjugate of the four-branched water-soluble polymer for medical use and two biologically relevant materials may have improved pharmacological activity as compared with conventional conjugates of a water-soluble polymer and one biologically relevant material.

This application is based on a patent application No. 2021-191074 filed in Japan, the contents of which are incorporated in full herein.

## Claims

1. A four-branched water-soluble polymer for medical use, represented by the formula (1) or the formula (2): 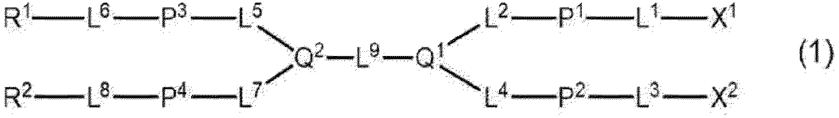 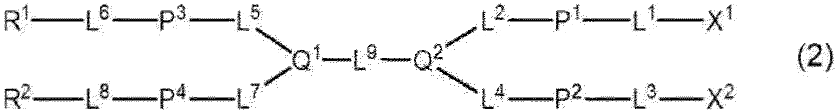 wherein
X¹ and X² are each independently a functional group capable of reacting with a biologically relevant material,
P¹ to P⁴ are each independently a divalent group of a water-soluble polymer,
Q¹ is a trivalent group represented by the formula (q1): 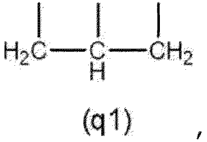
Q² is a trivalent group represented by any of the formula (q1) to the formula (q3): 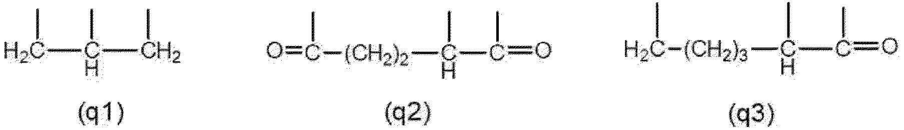
L¹ to L⁸ are each independently a single bond or a divalent spacer,
L⁹ is a divalent spacer, and
R¹ and R² are each independently a hydrocarbon group.

2. A four-branched water-soluble polymer for medical use, represented by the formula (3) or the formula (4): 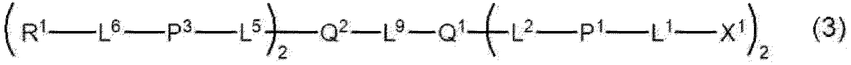 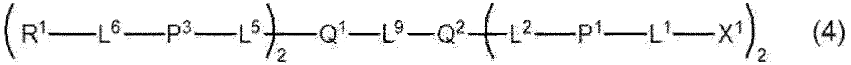 wherein
X¹ is a functional group capable of reacting with a biologically relevant material,
P¹ and P³ are each independently a divalent group of a water-soluble polymer,
Q¹ is a trivalent group represented by the formula (q1): 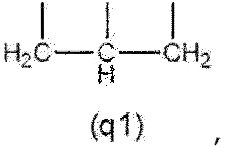
Q² is a trivalent group represented by any of the formula (q1) to the formula (q3): 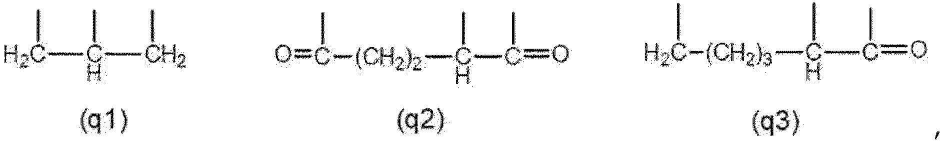
L¹, L², L⁵, and L⁶ are each independently a single bond or a divalent spacer,
L⁹ is a divalent spacer, and
R¹ is a hydrocarbon group.

3. The four-branched water-soluble polymer for medical use according to claim 1 or 2, wherein the divalent groups of the water-soluble polymers are each independently a divalent group of polyethylene glycol, polyoxazoline, or polysialic acid.

4. The four-branched water-soluble polymer for medical use according to any one of claims 1 to 3, wherein a total of the number average molecular weight of the divalent groups of the water-soluble polymers is not less than 4,000 and not more than 160,000.

5. The four-branched water-soluble polymer for medical use according to any one of claims 1 to 4, wherein the functional group capable of reacting with a biologically relevant material is an active ester group, an active carbonate group, a formyl group, an isocyanate group, an isothiocyanate group, an epoxy group, a maleimidyl group, a vinylsulfonyl group, an acryloyl group, an alkylsulfonyloxy group, a carboxy group, a mercapto group, a pyridyldithio group, an α-haloacetyl group, an alkynyl group, an allyl group, a vinyl group, an amino group, an aminooxy group, a hydrazide group, or an azido group.
